(19) Europäisches Patentamt / European Patent Office / Office européen des brevets

(11) EP 4 268 650 A1

(12) **EUROPEAN PATENT APPLICATION**
published in accordance with Art. 153(4) EPC

(43) Date of publication:
**01.11.2023 Bulletin 2023/44**

(21) Application number: **21910817.2**

(22) Date of filing: **21.12.2021**

(51) International Patent Classification (IPC):
***A41G 3/00*** *(2006.01)* ***D06M 13/12*** *(2006.01)*
***D06M 15/423*** *(2006.01)* ***D06M 15/643*** *(2006.01)*

(52) Cooperative Patent Classification (CPC):
**A41G 3/00; A61K 8/33; A61K 8/49; A61Q 5/06; D06M 13/12; D06M 15/423; D06M 15/643**

(86) International application number:
**PCT/JP2021/047434**

(87) International publication number:
**WO 2022/138677 (30.06.2022 Gazette 2022/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.12.2020 JP 2020217990**

(71) Applicant: **Kao Corporation**
**Chuo-ku**
**Tokyo 103-8210 (JP)**

(72) Inventor: **FURUKAWA, Junichi**
**Tokyo 131-8501 (JP)**

(74) Representative: **Hoffmann Eitle**
**Patent- und Rechtsanwälte PartmbB**
**Arabellastraße 30**
**81925 München (DE)**

(54) **FIBER TREATMENT AGENT**

(57) A fiber-treating agent for treating human hair fibers separated from human heads, the fiber-treating agent comprises a condensate formed from components (A) and (B), and a component (C), and has a turbidity of 1,000 NTU or less. (A): formaldehyde or a hydrate thereof; (B): a triazine derivative of formula (1) [wherein $R^1$ to $R^3$ are the same or different, and each represent a hydrogen atom, a hydroxymethylamino group, an amino group, a hydroxy group, a halogen atom, a phenyl group, a linear or branched alkyl group or alkenyl group having 1 or more and 6 or less carbon atoms, or a linear or branched alkoxy group or alkenyloxy group having 1 or more and 6 or less carbon atoms]; and (C): water.

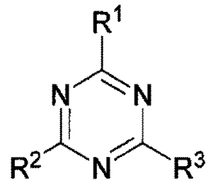

**(1)**



Processed by Luminess, 75001 PARIS (FR)

## Description

Field of the Invention

The present invention relates to a fiber-treating agent for semi-permanently or permanently changing the shape of human hair fibers as an industrial material.

Background of the Invention

Heretofore, as fibers implanted in hair ornament products such as wigs, those using artificial hair and those using human hair have been widely known.

The advantage of a hair ornament product using artificial hair is that the heat resistance is high, and thus it is possible to easily curl straight hair or straighten curled hair by a thermal appliance such as a curling iron (hereinafter, referred to as "shaping property") and that a curl- or straight-shape formed by a thermal appliance is sustained even under high-temperature and high-humidity conditions as in bathing, hair washing or the like (hereinafter, referred to as "shape sustainability"). Further, a hair ornament product using artificial hair is superior to human hair in that it is rigid and has high strength. On the other hand, a hair ornament product using artificial hair has a disadvantage, that is, it has a smooth surface, and hence high sleekness, and has an unnatural texture, so that it is difficult to achieve the hair ornament product's original purpose of pretending to wear natural hair.

The advantage of a hair ornament product using human hair is that it has similar in texture and sleekness to real hair, and gives a natural appearance when worn. Since most of people who wear a hair ornament product do not want others to know that they are wearing a hair ornament product, giving a natural appearance is a major advantage.

However, even a hair ornament product using human hair has a disadvantage. It is known that hair used for a hair ornament product undergoes a large number of treatment processes including removal of cuticles by chemical treatment in the beginning, bleaching, dyeing and the like for imparting a uniform shape and color, and may be damaged during the processes (Patent Literature 1), and these processes may cause an issue in durability (Patent Literature 2). In recent years, with a growing sense of beauty, the number of people who place importance on the visual appearance has increased, and the number of people who enjoy various hair styles with one hair ornament product has come to increase. For human hair, it is possible to easily curl straight hair or straighten curled hair by a thermal appliance such as a curling iron (having a shaping property), but a curl- or straight-shape formed by a thermal appliance is not maintained under high-temperature and high-humidity conditions as in bathing, hair washing or the like (having less shape sustainability), and for achieving such semi-permanent or permanent deformation, special chemical treatment such as permanent waving is required each time.

For compensating for such a disadvantage of human hair, for example, Patent Literature 1 discloses a fiber bundle for hair in which human hair and polyester-based fibers having specific physical properties are mixed to overcome the disadvantage of human hair poor in shape sustainability without impairing excellent characteristic of human hair. Patent Literature 2 discloses a hair ornament product in which accumulation of frizz which is a defect of a human hair product is improved, and the hair ornament product has permanent waving performance which is a defect of regenerated collagen fibers by blending regenerated collagen fibers and human hair.

Citation List

Patent Literatures

Patent Literature 1: WO 2005/037000
Patent Literature 2: JP-A-2007-177370
Patent Literature 3: JP-A-2019-143282

Summary of the Invention

The present invention provides a fiber-treating agent for treating human hair fibers separated from human heads, wherein the fiber-treating agent comprises a condensate formed from the following components (A) and (B), and a component (C), and has a turbidity of 1,000 NTU or less:

(A): formaldehyde or a hydrate thereof;
(B): a triazine derivative of formula (1):

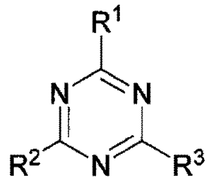

**(1)**

wherein $R^1$ to $R^3$ are the same or different, and each represent a hydrogen atom, a hydroxymethylamino group, an amino group, a hydroxy group, a halogen atom, a phenyl group, a linear or branched alkyl group or alkenyl group having 1 or more and 6 or less carbon atoms, or a linear or branched alkoxy group or alkenyloxy group having 1 or more and 6 or less carbon atoms; and

(C): water.

Further, the present invention provides a method for producing a fiber-treating agent for treating human hair fibers separated from human heads, the method comprising heating a composition until its turbidity becomes 1,000 NTU or less, the composition comprising the components (A) to (C).

Further, the present invention provides a method for treating fibers, comprising the following step (i):

(i) immersing human hair fibers separated from human heads in the fiber-treating agent to perform treatment while maintaining a state in which the turbidity of the treating agent is 1,000 NTU or less.

Further, the present invention provides a method for producing human hair fibers for hair ornament products, comprising the step of treating human hair fibers by the method for treating fibers.

Further, the present invention provides a method for producing a hair ornament product, comprising the step of treating human hair fibers by the method for treating fibers.

Further, the present invention provides a human hair fiber for hair ornament products, comprising a condensate formed from the components (A) and (B).

Further, the present invention provides a hair ornament product having, as a constituent element, human hair fibers containing a condensate formed from the components (A) and (B).

Detailed Description of the Invention

In the techniques disclosed in Patent Literatures 1 and 2, there still exists the disadvantage of being visually unnatural because human hair and artificial hair are mixed, and it cannot be said that those techniques are sufficient in terms of natural appearance.

For solving such a problem, Patent Literature 3 discloses a fiber-treating agent for producing human hair fibers for wigs which are excellent in shape sustainability and durability (elastic modulus) while maintaining the natural appearance of human hair, the fiber-treating agent comprising formaldehyde and a melamine derivative, and a method for treating human hair fibers.

However, in some situations of production of hair ornament products, hair is strongly stretched, and in the technique disclosed in Patent Literature 3, there are cases where the stretchability (tenacity) of treated human hair fibers is not sufficient. For this reason, it is required to further enhance the stretchability of treated human hair fibers for preventing rupture during extension.

Accordingly, the present invention relates to a fiber-treating agent for producing human hair fibers for producing human hair fibers for hair ornament products which are excellent in shape sustainability and tensile elastic modulus and maintains the stretchability (tenacity) of human hair fibers at a higher level while maintaining the natural appearance of human hair, and a method for treating fibers.

The present inventor found that when the fiber-treating agent disclosed in Patent Literature 3 is handled on the basis of the method for treating human hair fibers in this document, a composition in which the human hair fibers are immersed is changed through three-step phases. That is, in the phase 1, the composition has a high turbidity with formaldehyde and the melamine derivative being present while reacting independently of each other. In the phase 2, formaldehyde and the melamine derivative react with each other to form a water-soluble condensate, so that the composition becomes transparent. In the phase 3, the water-soluble condensates are further linked to each other to form a water-insoluble condensate, so that the turbidity of the composition increases again.

The present inventor found that when human hair fibers are brought into contact with the composition in the phase 1, free formaldehyde is directly applied to the human hair fibers to cross-link the tissues, so that it is difficult to maintain the stretchability (tenacity) of the human hair fibers. On the other hand, when human hair fibers are brought into contact with the composition in the phase 3, a hard resin layer formed on the surfaces of the human hair fibers, and

thus motions, such as bending and stretching, of the human hair fibers are restricted, so that it is also difficult to maintain the stretchability (tenacity) of the human hair fibers, and further, the feel of the human hair fiber surfaces is deteriorated.

The present inventor has further conducted studies on the basis of the above-described findings, and resultantly found that by treating human hair fibers separated from the head of a human using a composition comprising a condensate of formaldehyde and a specific triazine derivative and having a turbidity of 1,000 NTU or less while maintaining a state in which the turbidity of the treating agent is 1,000 NTU or less, not only shape sustainability is imparted to human hair fibers and the tensile elastic modulus is improved, but also it is possible to maintain high stretchability (tenacity), and improve the feel of the human hair fiber surfaces, leading to completion of the invention.

According to the present invention, it is possible to provide a fiber-treating agent for producing human hair fibers for hair ornament products which are excellent in shape sustainability and tensile elastic modulus and maintain the original stretchability (tenacity) of human hair fibers at a high level while maintaining the natural appearance of human hair.

[Condensate formed from components (A) and (B)]

The fiber-treating agent of the present invention comprises a condensate of formaldehyde or a hydrate thereof as a component (A), and a triazine derivative of formula (1) as a component (B). The condensate may include not only the above-described water-soluble condensate, but also a water-insoluble condensate formed by linkage of the water-soluble condensates as long as the turbidity of the fiber-treating agent is 1,000 NTU or less. Herein, the condensate includes both water-soluble and water-insoluble condensates when referred to simply as a "condensate". In the fiber-treating agent of the present invention, the unreacted component (A) and component (B) may remain in addition to such condensates as long as the turbidity is 1,000 NTU or less.

(Component (A): formaldehyde or hydrate thereof)

Examples of the hydrate of formaldehyde include formaldehyde monohydrate (methanediol). Among them, formaldehyde is preferable from the viewpoint of imparting higher shape sustainability and durability to treated human hair fibers.

The content of a constituent element derived from the component (A) in the fiber-treating agent of the present invention is preferably 0.1 mass% or more, more preferably 1 mass% or more, further more preferably 2.5 mass% or more, even more preferably 5 mass% or more, from the viewpoint of imparting higher shape sustainability and strength to treated human hair fibers, and preferably 60 mass% or less, more preferably 50 mass% or less, further more preferably 40 mass% or less, even more preferably 35 mass% or less, even more preferably 30 mass% or less, from the viewpoint of formulation compatibility in addition to the above-described viewpoint.

That is, the content of a constituent element derived from the component (A) in the fiber-treating agent of the present invention is preferably from 0.1 to 60 mass%, more preferably from 1 to 50 mass%, further more preferably from 2.5 to 40 mass%, even more preferably from 5 to 35 mass%, even more preferably from 5 to 30 mass%, from the viewpoint of imparting higher shape sustainability and strength to treated human hair fibers and the viewpoint of formulation compatibility.

Herein, the term "constituent element derived from the component (A)" refers to the total content of a constituent derived from the component (A) in the condensate and the remaining component (A).

(Component (B): triazine derivative of formula (1))

The triazine derivative for use in the present invention is represented by the following formula (1).

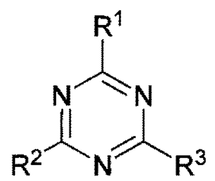

**(1)**

wherein $R^1$ to $R^3$ are the same or different, and each represent a hydrogen atom, a hydroxymethylamino group, an amino group, a hydroxy group, a halogen atom, a phenyl group, a linear or branched alkyl group or alkenyl group having 1 or more and 6 or less carbon atoms, or a linear or branched alkoxy group or alkenyloxy group having 1 or more and 6 or less carbon atoms.

Examples of the triazine derivative of formula (1) include melamine, monomethylol melamine, dimethylol melamine, trimethylol melamine, benzoguanamine, acetoguanamine, 2,4-diamino-1,3,5-triazine, ammeline and 2-chloro-4,6-diamino-1,3,5-triazine. From the viewpoint of imparting higher shape sustainability and durability to treated human hair fibers, at least one selected from the group consisting of melamine, monomethylol melamine, dimethylol melamine and trimethylol melamine is preferable, and melamine is more preferable. One component (B) may be used alone, or two or more components (B) may be used in combination.

The content of a constituent element derived from the component (B) in the fiber-treating agent of the present invention is preferably 0.1 mass% or more, more preferably 1 mass% or more, further more preferably 2.5 mass% or more, even more preferably 5 mass% or more, from the viewpoint of imparting higher shape sustainability and strength to treated human hair fibers, and preferably 60 mass% or less, more preferably 50 mass% or less, further more preferably 40 mass% or less, even more preferably 35 mass% or less, even more preferably 30 mass% or less, from the viewpoint of improving the feel of human hair fiber surfaces in addition to the above-described viewpoint.

That is, the content of a constituent element derived from the component (B) in the fiber-treating agent of the present invention is preferably from 0.1 to 60 mass%, more preferably from 1 to 50 mass%, further more preferably from 2.5 to 40 mass%, even more preferably from 5 to 35 mass%, even more preferably 5 mass% or more and 30 mass% or less, from the viewpoint of imparting higher shape sustainability and strength to treated human hair fibers and the viewpoint of improving the feel of the human hair fiber surfaces.

Herein, the term "constituent element derived from the component (B)" refers to the total content of a constituent derived from the component (B) in the condensate and the remaining component (B).

The total content of a constituent element derived from the component (A) and a constituent element derived from the component (B) in the fiber-treating agent of the present invention is preferably more than 1 mass%, more preferably 2.5 mass% or more, further more preferably 5 mass% or more, even more preferably 10 mass% or more, from the viewpoint of imparting higher shape sustainability and strength to treated human hair fibers, and preferably 80 mass% or less, more preferably 70 mass% or less, further more preferably 60 mass% or less, even more preferably 50 mass% or less, even more preferably 40 mass% or less, from the viewpoint of improving the feel of the human hair fiber surfaces.

The molar ratio of a constituent element derived from the component (A) to a constituent element derived from the component (B) in the fiber-treating agent of the present invention, (A)/(B), is preferably 0.005 or more, more preferably 0.01 or more, further more preferably 0.05 or more, even more preferably 0.1 or more, from the viewpoint of further improving the shape sustainability and strength of treated human hair fibers by a condensate formed by condensation of water-soluble condensates in the human hair fibers and having a larger molecular weight, and preferably less than 5, more preferably 4 or less, further more preferably 3 or less, even more preferably 2 or less, from the viewpoint of a good feel.

That is, the molar ratio of a constituent element derived from the component (A) to a constituent element derived from the component (B), (A)/(B), is preferably 0.005 or more and less than 5, more preferably from 0.01 to 4, further more preferably 0.05 to 3, even more preferably from 0.1 to 2, from the viewpoint of further improving the shape sustainability and strength of treated human hair fibers by a condensate formed by condensation of water-soluble condensates in the human hair fibers and having a larger molecular weight and the viewpoint of a good feel.

The condensate of the component (A) and the component (B) can be formed in the composition by heating the composition containing the components (A) to (C). The composition containing the components (A) to (C) is present for reacting independently each other right after preparation, and when the composition is heated, both the components react with each other to form a water-soluble condensate.

The content of the condensate formed from the components (A) and (B) in the fiber-treating agent of the present invention is preferably more than 1 mass%, more preferably 1.5 mass% or more, further more preferably 2.5 mass% or more, even more preferably 5 mass% or more, even more preferably 10 mass% or more, from the viewpoint of imparting higher shape sustainability and strength to treated human hair fibers, and preferably 80 mass% or less, more preferably 70 mass% or less, further more preferably 60 mass% or less, even more preferably 50 mass% or less, even more preferably 40 mass% or less, from the viewpoint of improving the feel of the human hair fiber surfaces in addition to the above-described viewpoint.

[Component (C): water]

The fiber-treating agent of the present invention contains water as a medium. The content of the component (C) in the fiber-treating agent of the present invention is preferably 10 mass% or more, more preferably 20 mass% or more, further more preferably 30 mass% or more, even more preferably 40 mass% or more, and preferably 99 mass% or less, more preferably 97 mass% or less, further more preferably 95 mass% or less, even more preferably 90 mass% or less.

That is, the content of the component (C) in the fiber-treating agent of the present invention is preferably from 10 to 99 mass%, more preferably from 20 to 97 mass%, further more preferably from 30 to 95 mass%, even more

preferably from 40 to 90 mass%.

[Component (D): organic compound having a Hansen solubility parameter SP value of from 16 to 40 $Mpa^{1/2}$]

The water-soluble condensates formed from the components (A) and (B) are gradually linked to each other, so that the molecular weight increases, leading to a decrease in water solubility, and when an insoluble condensate having a large molecular weight is formed in the fiber-treating agent of the present invention, motions, such as bending and stretching, of human hair fibers may be restricted by a hard resin layer formed on the surfaces of the human hair fibers, resulting in not only impairment of the stretchability (tenacity) of the fibers but also deterioration of the feel of the fiber surfaces. It is preferable that the fiber-treating agent of the present invention contains an organic compound having a Hansen solubility parameter SP value of 16 $Mpa^{1/2}$ or more and 40 $Mpa^{1/2}$ or less (excluding organic salts and compounds having an aldehyde group and having a molecular weight of 150 or less) from the viewpoint of easily dissolving oligomeric condensation products of the components (A) and (B), which are formed in the process of reaction and cause an increase in turbidity, by preventing aggregation of the oligomeric condensation products. Since organic salts, which have charge, rapidly increase the turbidity when present in the system, and compounds having an aldehyde group, such as glutaraldehyde, rapidly increase the turbidity by cross-linking the triazine derivatives at multiple points, the organic salts and the compounds having an aldehyde group are excluded from the component (D).

In the present invention, the Hansen solubility parameter SP value refers to δTot ($Mpa^{1/2}$) calculated at 25°C in the DIY program using Software Package HSPiP 4th Edition 4.1.07 based on Hansen Solubility Parameters: A User's handbook, CRC Press, Boca Raton FL, 2007.

Examples of the organic compound having a Hansen solubility parameter SP value of 16 $Mpa^{1/2}$ or more and 40 $Mpa^{1/2}$ or less in the compound (D) include monohydric alcohols, dihydric alcohols, dihydric alcohol derivatives, polyhydric alcohols with a valence number of 3 or more, lactam, imidazolidinone, pyrimidinone, lactone, alkylene carbonate, and other general-purpose organic solvents whose SP value is within the above-described range.

Specific examples of the compound having a Hansen solubility parameter SP value of 16 $Mpa^{1/2}$ or more and 40 $Mpa^{1/2}$ or less are listed below. The parenthesized numerical value in each example is a SP value calculated by the above-described method.

- Examples of monohydric alcohol: ethanol (25.4), 1-propanol (22.9), isopropyl alcohol (22.3) and 1-butanol (22.9)
- Examples of dihydric alcohol: ethylene glycol (31.6), diethylene glycol (29.2), triethylene glycol (26.1), tetraethylene glycol (24.3), pentaethylene glycol (23.1), hexaethylene glycol (22.2), propylene glycol (31.7), 1-dipropylene glycol (26.0) and tripropylene glycol (23.4)
- Examples of dihydric alcohol derivative: dipropylene glycol monomethyl ether (21.1), dipropylene glycol dimethyl ether (17.8), dipropylene glycol diacetate (19.0) and dipropylene glycol monomethyl ether acetate (18.5)
- Examples of polyhydric alcohol with a valence number of 3 or more: glycerin (35.7) and sorbitol (35.8)
- Examples of lactam: 2-pyrrolidone (24.8) and N-methylpyrrolidone (22.0)
- Examples of imidazolidinone: urea of ethylene (28.5), 1,3-dimethyl-2-imidazolidinone (22.3), dihydroxymethylethyleneurea (31.3), N,N'-dihydroxymethyl-4,5-dihydroxyethyleneurea (34.7) and DMDM hydantoin (28.1)
- Examples of pyrimidinone: N,N'-dimethylpropyleneurea (21.3)
- Examples of lactone: γ-butyrolactone (24.6)
- Examples of alkylene carbonate: ethylene carbonate (29.2) and propylene carbonate (27.1)
- Examples of general-purpose organic solvent: DMF (N,N-dimethylformamide) (24.2), DMAc (N,N-dimethylacetamide) (23.0), DMSO (dimethylsulfoxide) (23.6), THF (tetrahydrofuran) (18.2), 1,4-dioxane (20.5) and acetonitrile (23.9)

Among them, compounds having a Hansen solubility parameter SP value of 35.8 $Mpa^{1/2}$ or less are preferable, compounds having a Hansen solubility parameter SP value of 34.7 $Mpa^{1/2}$ or less are more preferable, and compounds having a Hansen solubility parameter SP value of 29.2 $Mpa^{1/2}$ or less are further more preferable, from the viewpoint of easily dissolving oligomeric condensation products of the components (A) and (B), which are formed in the process of reaction and cause an increase in turbidity, by preventing aggregation of the oligomeric condensation products. From the same viewpoint, compounds having a Hansen solubility parameter SP value of 17.8 $Mpa^{1/2}$ or more are preferable, compounds having a Hansen solubility parameter SP value of 21.1 $Mpa^{1/2}$ or more are more preferable, and compounds having a Hansen solubility parameter SP value of 22.0 $Mpa^{1/2}$ or more are more preferable.

Among them, dihydric alcohols, lactam and imidazolidinone are preferable, and at least one selected from the group consisting of diethylene glycol (29.2), triethylene glycol (26.1), N-methylpyrrolidone (22.0), 1,3-dimethyl-2-imidazorizinone (22.3) and DMDM hydantoin (28.1) is more preferable.

Any one component (D) may be used alone, or two or more components (D) may be used in combination. The content of the component (D) in the fiber-treating agent of the present invention is preferably 10 mass% or more, more

preferably 15 mass% or more, further more preferably 25 mass% or more, from the viewpoint of maintaining longer a state in which the turbidity of the fiber-treating agent is 1,000 or less, and preferably 80 mass% or less, more preferably 60 mass% or less, further more preferably 45 mass% or less, from the viewpoint of efficiently carrying out a condensation reaction and imparting high shape sustainability and strength to treated human hair fibers by a condensate formed by linkage of water-soluble condensates in the human hair fibers and having a large molecular weight.

[Cationic surfactant]

The fiber-treating agent of the present invention may contain a cationic surfactant as long as the effects of the present invention are not impaired. The cationic surfactant is preferably a long chain monoalkyl quaternary ammonium salt having one alkyl group having 8 to 24 carbon atoms and three alkyl groups having 1 to 4 carbon atoms.
Preferably, at least one long chain monoalkyl quaternary ammonium surfactant is selected from the group consisting of compounds of the following formula:

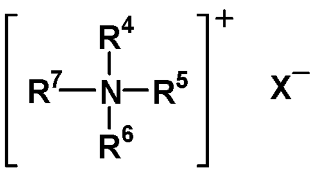

wherein $R^4$ is a saturated or unsaturated linear or branched alkyl group having 8 to 22 carbon atoms, $R^8$-CO-NH-$(CH_2)_m$- or $R^8$-CO-O-$(CH_2)_m$- ($R^8$ represents a saturated or unsaturated linear or branched alkyl chain having 7 to 21 carbon atoms, and m represents an integer of 1 to 4), $R^5$, $R^6$ and $R^7$ independently represent an alkyl group having 1 to 4 carbon atoms, or a hydroxyalkyl group having 1 to 4 carbon atoms, and $X^-$represents a hydrochloride ion, a bromide ion, a methosulfate ion or an ethosulfate ion.
Examples of the suitable cationic surfactant include long chain quaternary ammonium compounds such as cetyltrimethylammonium chloride, myristyltrimethylammonium chloride, behentrimonium chloride, cetyltrimethylammonium bromide and stearamidopropyltrimonium chloride. One of them may be used alone, or a mixture thereof may be used.
The content of the cationic surfactant in the fiber-treating agent of the present invention is preferably 0.05 mass% or more, more preferably 0.10 mass% or more, and preferably 10 mass% or less, more preferably 5 mass% or less, from the viewpoint of improving the feel of human hair fibers after treatment, and further improving the effects of the present invention.

[Silicone]

The fiber-treating agent of the present invention may contain silicone from the viewpoint of improving the feel of human hair fibers after treatment, and improving hair manageability. The silicone is preferably one or more selected from the group consisting of dimethylpolysiloxane and amino-modified silicone.
As the dimethylpolysiloxane, any of cyclic or acyclic dimethylsiloxane polymers can be used, and examples thereof include SH200 Series, BY22-019, BY22-020, BY11-026, B22-029, BY22-034, BY22-050A, BY22-055, BY22-060, BY22-083 and FZ-4188 (each manufactured by Dow Corning Toray), and KF-9088, KM-900 Series, MK-15H and MK-88 (each manufactured by Shin-Etsu Chemical Co., Ltd.).
As the amino-modified silicone, any silicone having an amino group or an ammonium group can be used, and examples thereof include amino-modified silicone oil which is terminal-blocked at all or a part of terminal hydroxyl groups with a methyl group or the like, and amodimethicone which is not terminal-blocked. Examples of the amino-modified silicone preferable from the viewpoint of improving the feel of human hair fibers after treatment and improving hair manageability include compounds of the following formula:

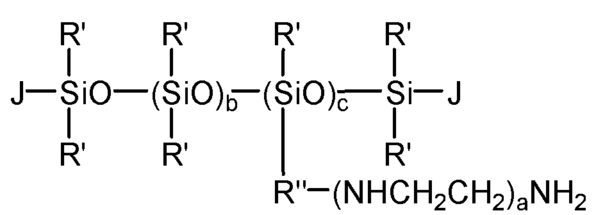

wherein R' represents a hydrogen atom, a hydroxy group or $R^X$, where $R^X$ represents a substituted or unsubstituted

monovalent hydrocarbon group having 1 to 20 carbon atoms, J represents $R^X$, R"-$(NHCH_2CH_2)aNH_2$, $OR^X$ or a hydroxy group, R" represents a divalent hydrocarbon group having 1 to 8 carbon atoms, a represents a number of 0 to 3, and b and c represent numbers whose sum is 10 or more and less than 20,000, preferably 20 or more and less than 3,000, more preferably 30 or more and less than 1,000, further more preferably 40 or more and less than 800, in terms of number average.

Specific examples of the suitable marketed product of amino-modified silicone include amino-modified silicone oils such as SF8452C and SS3551 (each manufactured by Dow Corning Toray) and KF-8004, KF-867S and KF-8015 (each manufactured by Shin-Etsu Chemical Co., Ltd.), and amodimethicone emulsions such as SM8704C, SM8904, BY22-079, FZ-4671 and FZ4672 (each manufactured by Dow Corning Toray).

The content of silicone in the fiber-treating agent of the present invention is preferably 0.1 mass% or more, more preferably 0.2 mass% or more, further more preferably 0.5 mass% or more, and preferably 20 mass% or less, more preferably 10 mass% or less, further more preferably 5 mass% or less, from the viewpoint of improving the feel of human hair fibers after treatment, and further improving the effects of the present invention.

[Cationic polymer]

The fiber-treating agent of the present invention may contain a cationic polymer from the viewpoint of improving the feel of human hair fibers after treatment.

The cationic polymer refers to a polymer having a cationic group, or a group capable of being ionized into a cationic group, and also includes a generally cationic ampholytic polymer. That is, examples of the cationic polymer include those in the form of an aqueous solution, which contain an amino group or an ammonium group on the side chain of the polymer chain or contain a diallyl quaternary ammonium salt as a constituent unit, for example, cationized cellulose derivatives, cationic starch, cationized guar gum derivatives, polymers or copolymers of a diallyl quaternary ammonium salt, and quaternized polyvinylpyrrolidone derivatives. Among them, one or more selected from the group consisting of a polymer containing a diallyl quaternary ammonium salt as a constituent unit, a quaternized polyvinylpyrrolidone derivative and a cationized cellulose derivative are preferable, and one or more selected from the group consisting of a polymer or copolymer of a diallyl quaternary ammonium salt and a cationized cellulose derivative are more preferable, from the viewpoint of improving the effects of softness, smoothness and finger-combability in the feel during rinsing and shampooing and hair manageability and moisture retainability during blowing, and the stability of the agent.

Specific examples of the suitable polymer or copolymer of a diallyl quaternary ammonium salt include dimethyldiallylammonium chloride polymers (polyquaternium-6, for example, MERQUAT 100; Lubrizol Advanced Materials, Inc.), dimethyldiallylammonium chloride/acrylic acid copolymers (polyquaternium-22, for example, MERQUATs 280 and 295; Lubrizol Advanced Materials, Inc.), and dimethyldiallylammonium chloride/acrylamide copolymers (polyquaternium-7, for example, MERQUAT 550; Lubrizol Advanced Materials, Inc.).

Specific examples of the suitable quaternized polyvinylpyrrolidone derivative include polymers obtained by polymerizing a vinylpyrrolidone copolymer and dimethylaminoethyl methacrylate (polyquaternium 11, for example, GAFQUAT 734, GAFQUAT 755 and GAFQUAT 755N (Ashland Inc.)).

Specific examples of the suitable cationized cellulose include polymers obtained by adding glycidyltrimethylammonium chloride to hydroxycellulose (polyquaternium 10, for example, LEOGARDs G and GP (Lion Corporation) and POLYMERs JR-125, JR-400, JR-30M, LR-400 and LR-30M (Amerchol Corporation)), and hydroxyethylcellulose dimethyldiallylammonium chloride (polyquaternium-4, for example, CELQUATs H-100 and L-200 (Akzo Nobel N.V.).

The content of the cationic polymer in the fiber-treating agent of the present invention is preferably 0.001 mass% or more, more preferably 0.01 mass% or more, further more preferably 0.05 mass% or more, and preferably 20 mass% or less, more preferably 10 mass% or less, from the viewpoint of improving the feel of human hair fibers after treatment.

Further, the fiber-treating agent of the present invention may contain an antioxidant such as ascorbic acid, and a pH adjuster such as sodium hydroxide, potassium hydroxide, phosphoric acid or hydrochloric acid.

[Turbidity]

It is desirable that the turbidity of the fiber-treating agent of the present invention be as low as possible from the viewpoint of maintaining high stretchability (tenacity) of human hair fibers and the viewpoint of improving the feel of the human hair fiber surfaces, and the turbidity of the fiber-treating agent is 1,000 NTU or less, preferably 500 NTU or less, more preferably 100 NTU or less, further more preferably 20 NTU or less. The turbidity of the fiber-treating agent refers to one from turbidness originating from the component (B) and a water-insoluble condensate formed by bonding between water-soluble condensates, and when other components causing turbidness are present, only turbidness caused by the component (B) and the water-insoluble condensate is taken into account. Here, the amount of the water-insoluble condensate can be determined by, for example, a derivatization-pyrolysis GC/MS method after filtration with a membrane filter having a pore diameter of 0.1 μm.

Here, NTU (nephelometric turbidity unit) is a unit of turbidity in a turbidimetric method using formazin as a turbidity standard solution. The turbidity of the fiber-treating agent can be measured at room temperature (25°C) with the fiber-treating agent directly placed in a measurement cell of a digital turbidimeter (manufactured by AS ONE Corporation/model: TB700).

[pH]

The pH of the fiber-treating agent of the present invention is preferably 6.0 or more, more preferably 6.5 or more, further more preferably 7.0 or more, from the viewpoint of improving infiltration into human hair fibers, and preferably 12.0 or less, more preferably 11.5 or less, further more preferably 11.0 or less, from the viewpoint of suppressing damage to human hair fibers. The pH in the present invention is a value at 25°C.

That is, the pH of the fiber-treating agent of the present invention is preferably from 6.0 to 12.0, more preferably from 6.5 to 11.5, further more preferably from 7.0 to 11.0, from the viewpoint of improving infiltration into human hair fibers and suppressing damage to human hair fibers.

[Method for producing fiber-treating agent]

The fiber-treating agent of the present invention can be produced by heating the composition containing the components (A) to (C) until the turbidity is 1,000 NTU or less. The composition containing the components

(A) to (C) has a high turbidity with the component (A) and the component
(B) being present for reacting independently each other, and when the composition is heated, both the components react with each other to form a water-soluble condensate, so that the turbidity of the composition decreases. The fiber-treating agent of the present invention is a composition in this state, which has a turbidity of 1,000 NTU or less.

The heating temperature for setting the turbidity of the composition containing the components (A) to (C) to 1,000 NTU or less is preferably 85°C or higher, more preferably 88°C or higher, further more preferably 90°C or higher, from the viewpoint of ensuring that a reaction between the component (A) and the component (B) in the fiber-treating agent efficiently proceeds to form a condensate, and preferably 120°C or lower, more preferably 115°C or lower, further more preferably 110°C or lower, from the viewpoint of preventing the reaction from becoming too fast to be controlled.

The heating time is preferably 1 minute or more, more preferably 5 minutes or more, and preferably 20 minutes or less, more preferably 15 minutes or less. It is desirable that the temperature be raised to the above-described temperature in a short time, and after confirmation of achievement of transparency by heating and stirring, cooling be performed in a short time to lower the temperature to a temperature for application to fibers or a temperature for storage of the fiber-treating agent as described later. When it is difficult to rapidly raise the temperature due to scale-up, it is advantageous that the temperature is raised with the composition mixed with the exclusion of the component (B), and the component (B) is added at the time of reaching the above-described temperature.

The pH of the composition containing the components (A) to (C) during heating is preferably 7.0 or more, more preferably 8.0 or more, further more preferably 9.0 or more, from the viewpoint of preventing the reaction from becoming excessively fast, resulting in entrance into the phase 3, and preferably 13.0 or less, more preferably 12.0 or less, further more preferably 11.0 or less, from the viewpoint of workability for easily adjusting the pH during application to human hair fibers. After the composition has a turbidity of 1,000 or less, the pH can be adjusted to be in the above-described suitable pH range of the fiber-treating agent as necessary.

[Method for storing fiber-treating agent]

When the treating agent produced as described above and having a turbidity of 1,000 NTU or less is transported and stored until application to human hair fibers, it is also possible to set the storage temperature to a cool temperature for the purpose of preventing unintended reaction progress during transportation. The storage temperature is preferably 1°C or higher, more preferably 2°C or higher, further more preferably 5°C or higher, from the viewpoint of preventing occurrence of freezing and recrystallization, and preferably 25°C or lower, more preferably 20°C or lower, further more preferably 15°C or lower, from the viewpoint of preventing progress of an unintentional reaction. It is preferable that the pH of the fiber-treating agent during storage be adjusted to be within the range of from 9 to 11 under which the condensation rate is low.

[Method for treating fibers]

(Basic treatment)

When using the fiber-treating agent of the present invention, human hair fibers are treated by a method comprising the following step (i), it is possible to impart shape sustainability and high durability to the human hair fibers while maintaining high stretchability (tenacity) of the human hair fibers.

(i) immersing human hair fibers separated from human heads in the fiber-treating agent of the present invention to perform treatment while maintaining a state in which the turbidity of the treating agent is 1,000 NTU or less.

The fiber-treating agent of the present invention produced as described above and having a turbidity of 1,000 NTU or less may be directly applied to human hair fibers, but when the fiber-treating agent is applied to human hair fibers after being heated for a certain time within the bounds of not causing an increase in turbidity with the treating agent entering the phase 3, the stretchability of the human hair fibers can be further enhanced. Thus, it is preferable to carry out the following step (0) before the step (i).

(0) heating fiber-treating agent

The heating treatment in the step (0) is preferably 40°C or higher, more preferably 60°C or higher, further more preferably 70°C or higher, from the viewpoint of improvement of productivity, and preferably 120°C or lower, more preferably 105°C or lower, further more preferably 99°C or lower, from the viewpoint of being able to stop heating at an appropriate point.

Assuming that T is a heating time for heating the treating agent having a turbidity of 1,000 NTU or less immediately after preparation until the turbidity exceeds 1,000 NTU again with the treating agent entering the phase 3, the heating time in the step (0) is preferably 0.2T or more, more preferably 0.3T or more, further more preferably 0.4T or more, from the viewpoint of exhibiting the effect of stretchability of human hair fibers, and preferably 0.8T or less, more preferably 0.7T or less, further more preferably 0.6T or less, form the viewpoint of securing a time until the turbidity exceeds 1,000 NTU with the treating agent entering the phase 3, that is, securing a time which enables the human hair fibers to be treated.

In the step (i), the human hair fibers immersed in the fiber-treating agent may be dry or wet. The amount of the fiber-treating agent in which the human hair fibers are immersed is preferably 2 or more, more preferably 3 or more, further more preferably 5 or more, even more preferably 10 or more, even more preferably 20 or more, and preferably 500 or less, more preferably 250 or less, further more preferably 100 or less, in terms of bath ratio to the mass of the human hair fibers (mass of fiber-treating agent/mass of human hair fibers).

That is, the bath ratio is preferably from 2 to 500, more preferably from 3 to 250, further more preferably from 5 to 100, even more preferably from 10 to 100, even more preferably from 20 to 100.

In the step (i), the human hair fibers may be fixed with a curler or the like, followed by immersion in the fiber-treating agent of the present invention under heating. This enables a desired shape to be imparted to the human hair fibers together with shape sustainability and high durability.

The immersion of the human hair fibers in the fiber-treating agent in the step (i) is carried out under heating, and this heating is performed by heating the fiber-treating agent. This heating may be performed by immersing the human hair fibers in the fiber-treating agent being heated, or by immersing the human hair fibers in the fiber-treating agent at a low temperature, and then performing heating. The temperature of the fiber-treating agent is preferably 30°C or higher, more preferably 40°C or higher, further more preferably 50°C or higher for increasing interaction between the water-soluble condensate formed from the component (A) and the component (B) and the protein in human hair fibers, and accelerating a condensation/growth reaction between the water-soluble condensates formed from the component (A) and the component (B) in human hair fibers to obtain the effects of the present invention, and preferably lower than 95°C, more preferably 85°C or lower, further more preferably 75°C or lower, for suppressing thermal damage to human hair fibers.

Assuming that T is a heating time until the treating agent has a turbidity of more than 1,000 NTU after the treating agent having a turbidity of 1,000 NTU or less is heated immediately after preparation, the immersion time in the step (i) is preferably 0.3T or more, more preferably 0.4T or more, further more preferably 0.45T or more, from the viewpoint of exhibiting a stretchability improving effect on human hair fibers, and preferably 0.95T or less, more preferably 0.90T or less, further more preferably 0.85T or less for suppressing damage to human hair fibers.

The specific immersion time is appropriately adjusted depending on a heating temperature used, and is preferably 15 minutes or more, more preferably 30 minutes or more, further more preferably 1 hour or more, from the viewpoint of exhibiting a stretchability improving effect on human hair fibers, and preferably 48 hours or less, more preferably 24 hours or less, further more preferably 12 hours or less for suppressing damage to human hair fibers, for

example.

It is preferable to carry out the step (i) in an environment where evaporation of moisture is suppressed. Examples of the specific means for suppressing evaporation of moisture include a method in which a container of the fiber-treating agent in which human hair fibers are immersed is covered with a film-shaped material, a cap, a lid or the like made of a material impermeable to water vapor.

After the step (i), the human hair fibers may be rinsed, or may not be necessarily rinsed, and it is preferable to rinse the human hair fibers from the viewpoint of preventing deterioration of the feel of the human hair fibers by an excess polymerized product.

These treatments may allow the water-soluble condensate formed from the components (A) and (B) to infiltrate the human hair fibers and interact with protein in the human hair fibers. In the human hair fibers, the water-soluble condensates are fused together to form a condensate having a larger molecular weight. Since such a condensate remains in the human hair fibers even after the human hair fibers are washed, the human hair fibers treated by the method of the present invention do not lose shape even when washed.

Since if the turbidity of the treating agent increases during treatment in the step (i), a hard resin layer is formed on the surfaces of human hair fibers, so that it is difficult to maintain high stretchability (tenacity) of the human hair fibers, and it is difficult to secure a good feel of the human hair fiber surfaces, it is preferable to take out the human hair fibers from the treating agent before the turbidity of the treating agent exceeds 1,000 NTU. The turbidity of the treating agent can be confirmed by the above-described turbidity measurement method with a sample appropriately taken from the treating agent. If the human hair fibers taken out during treatment have not been sufficiently treated, the step (i) may be carried out again. That is, it is preferable to carry out step (ii) after the step (i), and to repeat the step (i) and the step (ii) two or more times.

(ii) taking out human hair fibers from the treating agent before the turbidity of the treating agent exceeds 1,000 NTU.

It is preferable to wash out the insoluble condensate by rinsing the surfaces of human hair fibers after taking out the human hair fibers from the treating agent in the step (ii). That is, it is preferable to carry out the following step (iii) after the step (ii).

(iii) rinsing human hair fibers taken out

It is desirable that the rinsing in the step (iii) be performed using a composition containing the component (D). The rinsing composition may be composed only of the component (D), or may contain water in addition to the component (D). When water is contained, the content of the component (D) in the rinsing composition is preferably 60 mass% or more, more preferably 80 mass% or more, further more preferably 95 mass% or more.

(Post-cross-linking treatment)

After the step (i) to (iii), the following step (iv) may be performed which enables further improvement of the shape sustainability of human hair fibers:
(iv) immersing human hair fibers in a post-cross-linking agent containing components (E) and (C):

(E): at least one formaldehyde derivative selected from the group consisting of formaldehyde, a hydrate of formaldehyde, glyoxylic acid, a hydrate of glyoxylic acid, a glyoxylic acid salt, glyoxal, a hydrate of glyoxal, glutaraldehyde, and a hydrate of glutaraldehyde; and
(C): water.

The content of the component (E) in the post-cross-linking agent is preferably 0.01 mass% or more, more preferably 0.1 mass% or more, further more preferably 1 mass% or more, and preferably 60 mass% or less, more preferably 40 mass% or less, further more preferably 20 mass% or less.

That is, the content of the component (E) in the post-cross-linking agent is preferably from 0.01 to 60 mass%, more preferably from 0.1 to 40 mass%, further more preferably from 1 to 20 mass%.

Further, the post-cross-linking agent may contain a pH adjuster such as sodium hydroxide, potassium hydroxide, phosphoric acid, hydrochloric acid or an organic acid. On the other hand, from the viewpoint of improving the feel of the surfaces of human hair fibers, it is preferable that the post-cross-linking agent be free of a triazine derivative as the component (B) and a resorcin derivative as a component (F) described later.

The pH of the post-cross-linking agent is preferably 6.0 or less, more preferably 5.0 or less, further more preferably 4.5 or less, from the viewpoint of improving infiltration into human hair fibers, and preferably 0 or more, more preferably 0.5 or more, further more preferably 1.0 or more, from the viewpoint of suppressing damage to human hair fibers.

That is, the pH of the post-cross-linking agent is preferably from 0 to 6.0, more preferably from 0.5 to 5.0, further more preferably from 1.0 to 4.5, from the viewpoint of improving infiltration into human hair fibers and the viewpoint of

suppressing damage to human hair fibers.

The temperature of the post-cross-linking agent used in the step (iv) is preferably 40°C or higher, more preferably 50°C or higher, further more preferably 60°C or higher, from the viewpoint of increasing interaction between the condensate formed from the component (A) and the component (B) and the protein in human hair fibers to enhance the effects of the present invention (shape sustainability and strength), and preferably lower than 100°C, more preferably 99°C or lower, from the viewpoint of preventing a situation in which the treating agent heavily boils during heating, so that human hair fibers are entangled, leading to deterioration of workability in the subsequent step.

In the step (iv), the human hair fibers to be immersed in the post-cross-linking agent may be dry or wet. The amount of the post-cross-linking agent in which human hair fibers are immersed is preferably 2 or more, more preferably 3 or more, further more preferably 5 or more, even more preferably 10 or more, even more preferably 20 or more, and preferably 500 or less, more preferably 250 or less, further more preferably 100 or less, in terms of a bath ratio to the mass of the human hair fibers (mass of post-cross-linking agent/mass of human hair fibers treated in the steps (i) to (iii)).

That is, the bath ratio is preferably from 2 to 500, more preferably from 3 to 250, further more preferably from 5 to 100, even more preferably from 10 to 100, even more preferably from 20 to 100.

The time for immersion of the human hair fibers in the post-cross-linking agent in the step (iv) is preferably 1 minute or more, more preferably 3 minutes or more, further more preferably 5 minutes or more, and preferably 5 hours or less, more preferably 3 hours or less, further more preferably 1 hour or less, for infiltrating and diffusing the post-cross-linking agent into the human hair fibers.

(Surface finish treatment)

The following step (v) may be further carried out after the steps (i) to (iii) or after the step (iv), and by carrying out the step (v), the feel of the human hair fiber surfaces can be significantly improved:

(v) immersing human hair fibers in a surface finish agent (I) containing components (F) and (C):

(F): a resorcin derivative of formula (2):

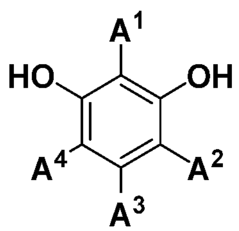

(2)

wherein

$A^1$ to $A^4$ are the same or different, and each represent a hydrogen atom, a hydroxy group, a halogen atom, a carboxyl group or a salt thereof, a sulfonic acid group or a salt thereof, a linear or branched alkyl group or alkenyl group having 1 to 6 carbon atoms, or a linear or branched alkoxy group or alkenyloxy group having 1 to 6 carbon atoms.

(C): water.

Examples of the preferred component (F) here include resorcin, 2-methylresorcin, 4-chlororesorcin, and pyrogallol. The content of the component (F) in the surface finish agent (I) is preferably 10 mass% or more, more preferably 20 mass% or more, further more preferably 30 mass% or more, even more preferably 40 mass% or more, even more preferably 50 mass% or more, and preferably 98 mass% or less, more preferably 97 mass% or less, further more preferably 95 mass% or less, even more preferably 90 mass% or less, even more preferably 80 mass% or less, from the viewpoint of improving the feel of the human hair fiber surfaces.

Further, the surface finish agent (I) may contain a pH adjuster such as sodium hydroxide, potassium hydroxide, phosphoric acid, hydrochloric acid or an organic acid. On the other hand, from the viewpoint of improving the feel of the human hair fiber surfaces, it is preferable that the surface finish agent (I) be free of a triazine derivative as the component (B) and a formaldehyde derivative as the component (E).

The pH of the surface finish agent (I) is preferably 7.0 or less, more preferably 6.8 or less, further more preferably 6.5 or less, from the viewpoint of improving infiltration into human hair fibers, and preferably 0 or more, more preferably 0.5 or more, further more preferably 1.0 or more, from the viewpoint of suppressing damage to human hair fibers.

That is, the pH of the surface finish agent (I) is preferably from 0 to 7.0, more preferably from 0.5 to 6.8, further more preferably from 1.0 to 6.5, from the viewpoint of improving infiltration into human hair fibers and the viewpoint of suppressing damage to human hair fibers.

The temperature of the surface finish agent (I) used in the step (v) is preferably 0°C or higher, preferably 20°C or higher, further more preferably 40°C or higher, and preferably 80°C or lower, more preferably 60°C or lower, from the viewpoint of efficiently infiltrating and diffusing the surface finish agent (I) into human hair fibers to further enhance the effect of improving the feel.

In the step (v), the human hair fibers to be immersed in the surface finish agent (I) may be dry or wet. The amount of the surface finish agent in which the human hair fibers are immersed is preferably 2 or more, more preferably 3 or more, further more preferably 5 or more, even more preferably 10 or more, even more preferably 20 or more, and preferably 500 or less, more preferably 250 or less, further more preferably 100 or less, in terms of a bath ratio to the mass of the human hair fibers (mass of surface finish agent (I)/mass of human hair fibers treated in the steps (i) to (iii) or the step (iv)).

That is, the bath ratio is preferably from 2 to 500, more preferably from 3 to 250, further more preferably from 5 to 100, even more preferably from 10 to 100, even more preferably from 20 to 100.

The time for immersion of the human hair fibers in the surface finish agent (I) in the step (v) is preferably 1 hour or more, more preferably 3 hours or more, further more preferably 6 hours or more, even more preferably 24 hours or more, and preferably 1 month or less, more preferably 2 weeks or less, further more preferably 10 days or less, even more preferably 168 hours or less, for infiltrating and diffusing the surface finish agent (I) into the human hair fibers.

[Optional additional treatments]

In the method for treating fibers according to the present invention, one or more treatments selected from the group consisting of removal of cuticles, bleaching, hair dyeing and surface finish for imparting hydrophobicity and reducing friction may be additionally performed in addition to the above-described steps (i) to (v).

Here, the treatments of removal of cuticles, bleaching and hair dyeing may be performed before or after the above-described steps (i) to (v), or may be performed between the steps (i) to (v). It is also possible to add a combination of a plurality of treatments, and when both bleaching and hair dyeing are added, any of the treatments may be performed first except that it is necessary to perform bleaching before hair dyeing, and it is also possible to perform another treatment such as removal of cuticles between bleaching and hair dyeing.

While, it is necessary that the surface finish for imparting hydrophobicity and reducing friction be performed after the above-described steps (i) to (iii), or after the step (iv) when treatment with the post-cross-linking agent in the step (iv) is also performed, or after the step (v) when surface finish for improving the feel in the step (v) is also performed, and in particular, by performing the surface finish for imparting hydrophobicity and reducing friction is additionally performed after the step (v), a better result can be obtained. As long as the surface finish for imparting hydrophobicity and reducing friction is performed after the steps (i) to (iii), after the step (iv) or after the step (v) as described above, the treatment order relation with removal of cuticles, bleaching and hair dyeing is not particularly limited.

(Removal of cuticles)

Cuticles present on the human hair fiber surface have a directional structure like scales lying on top of one another, and therefore if human hair fibers having different directionalities exist in one fiber bundle, the fibers are easily caught by each other and entangled, so that workability is significantly impaired. For this reason, removal of cuticles is conducted for eliminating the directionality of human hair fibers to facilitate handling, and performed by immersing human hair fibers in the following cuticle removing composition.

The cuticle removing composition contains a chlorine precursor having a cuticle peeling action, and water. Examples of the chlorine precursor include sodium hypochlorite, potassium dichloroisocyanurate, sodium dichloroisocyanurate, and trichloroisocyanuric acid, and one or more chlorine precursors selected from these compounds can be used.

The content of the chlorine precursor in the cuticle removing composition is preferably 1 mass% or more, more preferably 2 mass% or more, and preferably 15 mass% or less, more preferably 12 mass% or less, further more preferably 9 mass% or less.

The pH of the cuticle removing composition at 25°C is preferably 2.0 or more, more preferably 3.0 or more, further more preferably 4.5 or more, and preferably 10 or less, more preferably 8.0 or less, further more preferably 7.0 or less.

(Bleaching)

The bleaching is performed by immersing human hair fibers in a bleach composition containing an alkali agent, an oxidizing agent and water. The bleach composition is typically of two-part type. The first part contains an alkali agent and water, and the second part contains an oxidizing agent and water. These two parts are typically stored separately,

and mixed before immersion of human hair fibers.

Examples of the suitable alkali agent include, but are not limited to, ammonia and salts thereof; alkanolamines (monoethanolamine, isopropanolamine, 2-amino-2-methylpropanol, 2-aminobutanol and the like) and salts thereof; alkanediamines (1,3-propanediamine and the like) and salts thereof; carbonates (guanidine carbonate, sodium carbonate, potassium carbonate, sodium hydrogen carbonate, potassium hydrogen carbonate and the like); and mixtures thereof.

The content of the alkali agent in the bleach composition (mixture of first part and second part for two-part type) is preferably 0.1 mass% or more, more preferably 0.5 mass% or more, further more preferably 1 mass% or more, and preferably 15 mass% or less, more preferably 10 mass% or less, further more preferably 7.5 mass% or less).

Examples of the suitable oxidizing agent include, but are not limited to, hydrogen peroxide, urea peroxide, melamine peroxide and sodium bromate. Among these oxidizing agents, hydrogen peroxide is preferable.

The content of the oxidizing agent in the bleach composition is preferably 1 mass% or more, more preferably 2 mass% or more, and preferably 15 mass% or less, more preferably 12 mass% or less, further more preferably 9 mass% or less.

When the first part and the second part are stored separately, the pH of the second part at 25°C is preferably 2 or more, more preferably 2.5 or more, and preferably 6 or less, more preferably 4 or less. The pH can be adjusted by a suitable buffering agent. The pH of the bleach composition at 25°C is preferably 6 or more, more preferably 6.5 or more, further more preferably 6.8 or more, and preferably 11 or less, more preferably 10.5 or less, further more preferably 10 or less.

(Hair dyeing)

The hair dyeing is performed by immersing human hair fibers in a hair dye composition. The hair dye composition contains a dye, and optionally contains an alkali agent or an acid, an oxidizing agent and the like. Examples of the dye include direct dyes, oxidizing dyes, and combinations thereof.

The type of the direct dye is not particularly limited, and any direct dye suitable for hair dyeing can be used. Examples of the direct dye include anionic dyes, nitro dyes, disperse dyes, cationic dyes, and dyes having an azo-phenol structure selected from the group consisting of the following HC Red 18, HC Blue 18 and HC Yellow 16, salts thereof, and mixtures thereof.

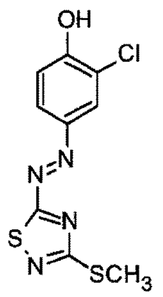

HC Red 18

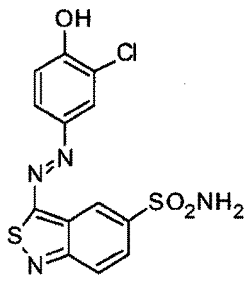

HC Blue 18

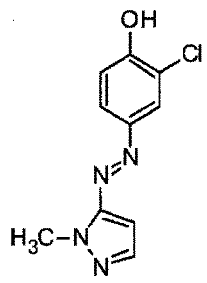

HC Yellow 16

Examples of the cationic dye include, but are not limited to, Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Orange 31, Basic Red 2, Basic Red 12, Basic Red 22, Basic Red 51, Basic Red 76, Basic Violet 1, Basic Violet 2, Basic Violet 3, Basic Violet 10, Basic Violet 14, Basic yellow 57, Basic Yellow 87, and mixtures thereof. Basic Red 51, Basic Orange 31, Basin Yellow 87 and mixtures thereof are particularly preferable.

Examples of the anionic dye include, but are not limited to, Acid Black 1, Acid Blue 1, Acid Blue 3, Food Blue 5, Acid Blue 7, Acid Blue 9, Acid Blue 74, Acid Orange 3, Acid Orange 4, Acid Orange 6, Acid Orange 7, Acid Orange 10, Acid Red 1, Acid Red 14, Acid Red 18, Acid Red 27, Acid Red 33, Acid Red 50, Acid Red 52, Acid Red 73, Acid Red 87, Acid Red 88, Acid Red 92, Acid Red 155, Acid Red 180, Acid Violet 2, Acid Violet 9, Acid Violet 43, Acid Violet 49, Acid Yellow 1, Acid Yellow 10, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, alkali metal salts (sodium salts, potassium salts and the like), and mixtures thereof.

Among them, preferred anionic dyes are Acid Black 1, Acid Red 52, Acid Violet 2, Acid Violet 43, Acid Red 33, Acid Orange 4, Acid Orange 7, Acid Red 27, Acid Yellow 3, Acid Yellow 10, and salts thereof. More preferred anionic

dyes are Acid Red 52, Acid Violet 2, Acid red 33, Acid Orange 4, Acid Yellow 10, and salts and mixtures thereof.

Examples of the nitro dye include, but are not limited to, HC Blue No. 2, HC Blue No. 4, HC Blue No. 5, HC Blue No. 6, HC Blue No. 7, HC Blue No. 8, HC Blue No. 9, HC Blue No. 10, HC Blue No. 11, HC Blue No. 12, HC Blue No. 13, HC Brown No. 1, HC Brown No. 2, HC Green No. 1, HC Orange No. 1, HC Orange No. 2, HC Orange No. 3, HC Orange No. 5, HC Red BN, HC Red No. 1, HC Red No. 3, HC Red No. 7, HC Red No. 8, HC Red No. 9, HC Red No. 10, HC Red No. 11, HC Red No. 13, HC Red No. 54, HC Red No. 14, HC Violet BS, HC Violet No. 1, HC Violet No. 2, HC Yellow No. 2, HC Yellow No. 4, HC Yellow No. 5, HC Yellow No. 6, HC Yellow No. 7, HC Yellow No. 8, HC Yellow No. 9, HC Yellow No. 10, HC Yellow No. 11, HC Yellow No. 12, HC Yellow No. 13, HC Yellow No. 14, HC Yellow No. 15, 2-amino-6-chloro-4-nitrophenol, picramic acid, 1,2-diamino-4-nitrobenzol, 1,4-diamino-2-nitrobenzol, 3-nitro-4-aminophenol, 1-hydroxy-2-amino-3-nitrobenzol, 2-hydroxyethylepicramic acid, and mixtures thereof.

Examples of the disperse dye include, but are not limited to, Disperse Blue 1, Disperse Black 9, Disperse Violet 1, and mixtures thereof.

One of these direct dyes may be used alone, or two or more thereof may be used in combination. Direct dyes different in ionicity may be used in combination.

The content of the direct dye in the hair dye composition is preferably 0.001 mass% or more, more preferably 0.01 mass% or more, further more preferably 0.05 mass% or more, from the viewpoint of obtaining sufficient hair dyeability, and preferably 10 mass% or less, more preferably 7.5 mass% or less, further more preferably 5.0 mass% or less, further more preferably 3.0 mass% or less, from the viewpoint of compatibility.

When the hair dye composition contains only direct dyes as a dye, an oxidizing agent is not necessary for dyeing human hair fibers. When it is desirable that human hair fibers be light-colored, the composition may contain an oxidizing agent.

When the hair dye composition contains an oxidizing dye, the composition is typically of two-part type. The first part contains an oxidizing dye intermediate (precursor and coupler) and an alkali agent, and the second part contains an oxidizing agent such as hydrogen peroxide. These two parts are typically stored separately, and mixed before immersion of human hair fibers.

The oxidizing dye intermediate is not particularly limited, and it is possible to suitably use any of known precursors and couplers which are commonly used for dyed products.

Examples of the precursor include, but are not limited to, paraphenylenediamine, toluene-2,5-diamine, 2-chloroparaphenylenediamine, N-methoxyethyl-para-phenylenediamine, N-phenylparaphenylenediamine, N,N-bis(2-hydroxyethyl)-paraphenylenediamine, 2-(2-hydroxyethyl)-paraphenylenediamine, 2,6-dimethyl-paraphenylenediamine, 4,4'-diaminodiphenylamine, 1,3-bis(N-(2-hydroxyethyl)-N-(4-aminophenyl)amino)-2-propanol, PEG-3,3,2'-paraphenylenediamine, paraaminophenol, paramethylaminophenol, 3-methyl-4-aminophenol, 2-aminomethyl-4-aminophenol, 2-(2-hydroxyethylaminoethyl)-4-aminophenol, ortho-aminophenol, 2-amino-5-methylphenol, 2-amino-6-methylphenol, 2-amino-5-acetamidophenol, 3,4-diaminobenzoic acid, 5-aminosalicylic acid, 2,4,5,6-tetraaminopyrimidine, 2,5,6-triamino-4-hydroxypyrimidine, 4,5-diamino-1-(4'-chlorobenzyl)pyrazole, 4,5-diamino-1-hydroxyethylpyrazole, salts of these substances, and mixture thereof.

Examples of the coupler include, but are not limited to, metaphenylenediamine, 2,4-diaminophenoxyethanol, 2-amino-4-(2-hydroxyethylamino)anisole, 2,4-diamino-5-methylphenetole, 2,4-diamino-5-(2-hydroxyethoxy)toluene, 2,4-dimethoxy-1,3-diaminobenzene, 2,6-bis(2-hydroxyethylamino)toluene, 2,4-diamino-5-fluorotoluene, 1,3-bis(2,4-diaminophenoxy)propane, metaaminophenol, 2-methyl-5-aminophenol, 2-methyl-5-(2-hydroxyethylamino)phenol, 2,4-dichloro-3-aminophenol, 2-chloro-3-amino-6-methylphenol, 2-methyl-4-chloro-5-aminophenol, N-cyclopentyl-metaaminophenol, 2-methyl-4-methoxy-5-(2-hydroxyethylamino)phenol, 2-methyl-4-fluoro-5-aminophenol, paraaminoorthocresol, resorcin, 2-methylresorcin, 4-chlororesorcin, 1-naphthol, 1,5-dihydroxynaphthalene, 1,7-dihydroxynaphthalene, 2,7-dihydroxynaphthalene, 2-isopropyl-5-methylphenol, 4-hydroxyindole, 5-hydroxyindole, 6-hydroxyindole, 7-hydroxyindole, 6-hydroxybenzomorpholine, 3,4-methylenedioxyphenol, 2-bromo-4,5-methylenedioxyphenol, 3,4-methylenedioxyaniline, 1-(2-hydroxyethyl)amino-3,4-methylenedioxybenzene, 2,6-dihydroxy-3,4-dimethylpyridine, 2,6-dimethoxy-3,5-diaminopyridine, 2,3-diamino-6-methoxypyridine, 2-methylamino-3-amino-6-methoxypyridine, 2-amino-3-hydroxypyridine, 2,6-diaminopyridine, salts of these substances, and mixtures thereof.

The content of each of the precursor and the coupler in the hair dye composition is preferably 0.01 mass% or more, more preferably 0.1 mass% or more, and preferably 10 mass% or less, more preferably 7.5 mass% or less, further more preferably 5 mass% or less.

When the hair dye composition contains an oxidizing dye, the hair dye composition further contains an alkali agent. Examples of the suitable alkali agent include, but are not limited to, ammonia and salts thereof; alkanolamines (monoethanolamine, isopropanolamine, 2-amino-2-methylpropanol, 2-aminobutanol and the like) and salts thereof; alkanediamines (1,3-propanediamine and the like) and salts thereof; carbonates (guanidine carbonate, sodium carbonate, potassium carbonate, sodium hydrogen carbonate, potassium hydrogen carbonate and the like); and mixtures thereof.

The content of the alkali agent in the hair dye composition is preferably 0.1 mass% or more, more preferably 0.5 mass% or more, further more preferably 1 mass% or more, and preferably 15 mass% or less, more preferably 10

mass% or less, further more preferably 7.5 mass% or less.

The composition containing an oxidizing agent (second part) when the hair dye composition contains an oxidizing dye is stored separately from the composition containing an oxidizing agent (first part), and mixed before human hair fibers are immersed. Examples of the suitable oxidizing agent include, but are not limited to, hydrogen peroxide, urea peroxide, melamine peroxide and sodium brominate. Among these oxidizing agents, hydrogen peroxide is preferable.

The content of the oxidizing agent in the hair dye composition is preferably 1 mass% or more, more preferably 2 mass% or more, and preferably 15 mass% or less, more preferably 12 mass% or less, further more preferably 9 mass% or less.

When the first part and the second part are stored separately, the pH of the second part at 25°C is preferably 2 or more, more preferably 2.5 or more, and preferably 6 or less, more preferably 4 or less. The pH can be adjusted by a suitable buffering agent. The pH of the hair dye composition obtained by mixing the first part and the second part at 25°C is preferably 6 or more, more preferably 6.5 or more, further more preferably 6.8 or more, and preferably 11 or less, more preferably 10.5 or less, further more preferably 10 or less.

When the hair dye composition contains an oxidizing dye, the hair dye composition may further contain any of the direct dyes exemplified above.

Preferably, the hair dye composition may further contain the following surfactant, conditioning component and the like. Preferably, the hair dye composition can be in the form of solution, emulsion, cream, paste and mousse.

The temperature of the hair dye composition is preferably 0°C or higher, more preferably 10°C or higher, further more preferably 20°C or higher, and preferably 90°C or lower, more preferably 80°C or lower, form the viewpoint of efficiently infiltrating and diffusing the hair dye composition into human hair fibers to enhance the effect of dyeing.

(Surface finish for imparting hydrophobicity and reducing friction)

The surface finish for imparting hydrophobicity and reducing friction is performed by immersing human hair fibers in the following surface finish agent (II) after the above-described steps (i) to (iii), or after the step (iv) when treatment with a post-cross-linking agent in the step (iv) is performed, or after the step (v) when surface finish for improving the feel in the step (v) is performed.

The surface finish agent (II) contains the following components (G) and (C):

(G) epoxyaminosilane copolymer which is a reaction product of the following compounds (a) to (d):

(a) polysiloxane having at least two oxiranyl groups or oxetanyl groups;
(b) polyether having at least two oxiranyl groups or oxetanyl groups;
(c) aminopropyltrialkoxysilane; and
(d) a compound selected from the group consisting of the following primary and secondary amines:

- primary amine: methylamine, ethylamine, propyleneamine, ethanolamine, isopropylamine, butylamine, isobutylamine, hexylamine, dodecylamine, oleylamine, aniline, aminopropyltrimethylsilane, aminopropyltriethylsilane, aminomorpholine, aminopropyldiethylamine, benzylamine, naphthylamine, 3-amino-9-ethylcarbazole, 1-aminoheptafluorohexane and 2,2,3,3,4,4,5,5,6,6,7,7,8,8,8-pentadecafluoro-1-octaneamine; and
- secondary amine: methylethylamine, methyloctadecylamine, diethanolamine, dibenzylamine, dihexylamine, dicyclohexylamine, piperidine, pyrrolidine phthalimide and polymer amine.

(C) water.

[Component (G): epoxyaminosilane copolymer]

The epoxyaminosilane copolymer as the component (G) is a reaction product of the following compounds (a) to (d).

<Compounds (a) and (b)>

The compound (a) is polysiloxane having at least two oxiranyl groups or oxetanyl groups, and examples thereof include compounds of the following formula (5):

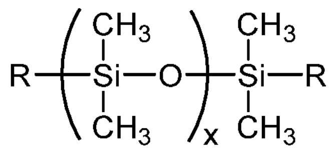

(5)

wherein R represents a hydrocarbon group having 1 to 6 carbon atoms and an oxiranyl group or an oxetanyl group at the terminal and optionally having a hetero atom, and x represents a number of 1 to 1,000.

The compound (b) is polyether having at least two oxiranyl groups or oxetanyl groups, and examples thereof include compounds of the following formula (6):

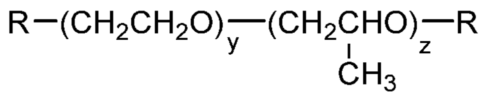

(6)

wherein R represents the same meaning as described above, y is 1 to 100, z is 0 to 100, and y + z represents a number of 1 to 200.

In formulae (5) and (6), the hetero atom optionally contained in R is preferably an oxygen atom. Examples of R include an oxiranylmethyl group (glycidyl group), an oxiranylmethoxy group (glycidyloxy group), an oxiranylmethoxypropyl group (glycidyloxypropyl group), an oxetanylmethyl group, an oxetanylmethoxy group, an oxetanylmethoxypropyl group and a 3-ethyloxetanylmethyl group. Among them, hydrocarbon groups having 1 to 4 carbon atoms and an oxiranyl group and optionally having a hetero oxygen atom are preferable, and at least one selected from the group consisting of an oxiranylmethyl group (glycidyl group), an oxiranylmethoxy group (glycidyloxy group) and an oxetanylmethyl group, an oxiranylmethoxypropyl group (glycidyloxypropyl group) is more preferable.

<Compound (c)>

The compound (c) is aminopropyltrialkoxysilane. Examples of the alkoxy group in the compound (c) include alkoxy groups having 1 to 6 carbon atoms, preferably 2 to 4 carbon atoms, more preferably 3 carbon atoms, and among them, an isopropoxy group is preferable. Examples of the compound (c) include aminopropyltrimethoxysilane, aminopropyltriethoxysilane, aminopropyltripropoxysilane, aminopropyltriisopropoxysilane, aminopropyltributoxysilane, and aminopropyltri-tert-butoxysilane, and among them, aminopropyltriisopropoxysilane is preferable. Any one compound (c) may be used alone, or two or more compounds (c) may be used in combination.

<Compound (d)>

The compound (d) is a compound selected from the group consisting of the following primary and secondary amines:

- primary amine: methylamine, ethylamine, propyleneamine, ethanolamine, isopropylamine, butylamine, isobutylamine, hexylamine, dodecylamine, oleylamine, aniline, aminopropyltrimethylsilane, aminopropyltriethylsilane, aminomorpholine, aminoethyldimethylamine, aminoethyldiethylamine, aminoethyldibutylamine, aminopropyldimethylamine, aminopropyldiethylamine, aminopropyldibutylamine, benzylamine, naphthylamine, 3-amino-9-ethylcarbazole, 1-aminoheptafluorohexane and 2,2,3,3,4,4,5,5, 6, 6,7,7, 8, 8, 8-pentadecafluoro-1-octane amine
- secondary amine: methylethylamine, methyloctadecylamine, diethanolamine, dibenzylamine, dihexylamine, dicyclohexylamine, piperidine, pyrrolidine phthalimide and polymer amine.

Among them, primary amines are preferable, and one selected from the group consisting of aminopropyldiethylamine, aminopropyldimethylamine and aminopropyldibutylamine is more preferable. One compound (d) may be used alone, or two or more compounds (d) may be used in combination.

The reaction of compounds (a) to (d) is carried out by, for example, refluxing the compounds in a solvent such as isopropanol for a certain period of time. Here, the molar ratio of oxiranyl groups or oxetanyl groups of compounds (a) and (b) to amino groups of the compound (c) is preferably 1 or more, more preferably 1.1 or more, further more preferably 1.2 or more, and preferably 4 or less, more preferably 3.9 or less, further more preferably 3.8 or less.

Examples of the component (G) include those having the INCI name of polysilicone-29, and examples of the marketed product thereof include Silsoft CLX-E (containing an active ingredient at 15 mass%, dipropylene glycol and water) from Momentive Performance Materials Company.

The content of the component (G) in the surface finish agent (II) is preferably 0.01 mass% or more, more

preferably 0.05 mass% or more, further more preferably 0.10 mass% or more, further more preferably 0.20 mass% or more, from the viewpoint of imparting sufficient hydrophobicity to human hair fibers, and preferably 15.00 mass% or less, more preferably 10.00 mass% or less, further more preferably 8.00 mass% or less, further more preferably 6.00 mass% or less, from the viewpoint that a sticky feel is not given.

[pH adjuster]

From the viewpoint of increasing the reaction rate of the trialkoxysilane part of the component (G) in an acidic region or a basic region, the pH of the surface finish agent (II) at 25°C is preferably in the following range. When the surface finish agent (II) is set as an acidic region, the pH is preferably 1.0 or more, more preferably 1.5 or more, further more preferably 2.0 or more, and preferably 5.0 or less, more preferably 4.0 or less, further more preferably 3.5 or less. When the surface finish agent (II) is set as a basic region, the pH is preferably 7.0 or more, more preferably 7.5 or more, further more preferably 8.0 or more, and preferably 11.0 or less, more preferably 10.5 or less, further more preferably 10.0 or less. The surface finish agent (II) may appropriately contain a pH adjuster for adjusting the pH of the surface finish agent (II) to be within the above-described range. As the pH adjuster, alkanol amines such as monoethanolamine, isopropanolamine, 2-amino-2-methylpropanol and 2-aminobutanol, or salts thereof; alkanediamines such as 1,3-propanediamine, or salts thereof; carbonates such as guanidine carbonate, sodium carbonate, potassium carbonate, sodium hydrogen carbonate and potassium hydrogen carbonate; hydroxides such as sodium hydroxide, potassium hydroxide; and the like can be used as the alkali agent. As the acid agent, inorganic acids such as hydrochloric acid and phosphoric acid, hydrochlorides such as monoethanolamine hydrochloride, phosphorates such as monopotassium dihydrogen phosphate and disodium monohydrogen phosphate, and organic acids such as lactic acid and malic acid, and the like can be used.

The amount of the surface finish agent (II) in which human hair fibers are immersed is preferably 2 or more, more preferably 5 or more, further more preferably 10 or more, and preferably 100 or less, more preferably 50 or less, further more preferably 20 or less, in terms of bath ratio to the mass of the human hair fibers (mass of surface finish agent (II)/mass of human hair fibers).

When human hair fibers are treated by the above method for treating fibers, the human hair fibers contain a condensate formed from the components (A) and (B), so that it is possible to produce human hair fibers for hair ornament products which are excellent in shape sustainability and tensile elastic modulus and maintain the original stretchability (tenacity) of human hair fibers at a high level while maintaining the natural appearance of human hair, and it is also possible to produce a hair ornament product using the fibers.

In the present invention, examples of the hair ornament product include hair wigs, wigs, weavings, hair extensions, blade hairs, hair accessories, and doll hairs.

Concerning the embodiments described above, preferred aspects of the present invention will be further disclosed below.

<1> A fiber-treating agent for treating human hair fibers separated from human heads, the fiber-treating agent comprises a condensate formed from the following components (A) and (B), and a component (C), and has a turbidity of 1,000 NTU or less:

(A): formaldehyde or a hydrate thereof;
(B): a triazine derivative of formula (1):

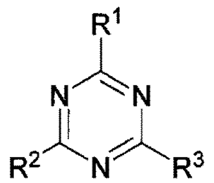

(1)

wherein $R^1$ to $R^3$ are the same or different, and each represent a hydrogen atom, a hydroxymethylamino group, an amino group, a hydroxy group, a halogen atom, a phenyl group, a linear or branched alkyl group or alkenyl group having 1 or more and 6 or less carbon atoms, or a linear or branched alkoxy group or alkenyloxy group having 1 or more and 6 or less carbon atoms; and
(C): water.

<2> The fiber-treating agent according to <1>, wherein a content of a constituent element derived from the component (A) in the fiber-treating agent is preferably 0.1 mass% or more, more preferably 1 mass% or more, further more

preferably 2.5 mass% or more, even more preferably 5 mass% or more, and preferably 60 mass% or less, more preferably 50 mass% or less, further more preferably 40 mass% or less, even more preferably 35 mass% or less, even more preferably 30 mass% or less.

<3> The fiber-treating agent according to <1> or <2>, wherein the component (B) is preferably at least one selected from the group consisting of melamine, monomethylol melamine, dimethylol melamine, trimethylol melamine, benzoguanamine, acetoguanamine, 2,4-diamino-1,3,5-triazine, ammeline and 2-chloro-4,6-diamino-1,3,5-triazine, more preferably at least one selected from the group consisting of melamine, monomethylol melamine, dimethylol melamine and trimethylol melamine, further more preferably melamine.

<4> The fiber-treating agent according to any one of <1> to <3>, wherein a content of a constituent element derived from the component (B) in the fiber-treating agent is preferably 0.1 mass% or more, more preferably 1 mass% or more, further more preferably 2.5 mass% or more, even more preferably 5 mass% or more, and preferably 60 mass% or less, more preferably 50 mass% or less, further more preferably 40 mass% or less, even more preferably 35 mass% or less, even more preferably 30 mass% or less.

<5> The fiber-treating agent according to any one of <1> to <4>, wherein a total of the content of a constituent element derived from the component (A) and the content of a constituent element derived from the component (B) in the fiber-treating agent is preferably more than 1 mass%, more preferably 2.5 mass% or more, further more preferably 5 mass% or more, even more preferably 10 mass% or more, and preferably 80 mass% or less, more preferably 70 mass% or less, further more preferably 60 mass% or less, even more preferably 50 mass% or less, even more preferably 40 mass% or less.

<6> The fiber-treating agent according to any one of <1> to <5>, wherein a molar ratio of the constituent element derived from the component (A) to the constituent element derived from the component (B), (A)/(B), is preferably 0.005 or more, more preferably 0.01 or more, further more preferably 0.05 or more, even more preferably 0.1 or more, and preferably less than 5, more preferably 4 or less, further more preferably 3 or less, even more preferably 2 or less.

<7> The fiber-treating agent according to any one of <1> to <6>, wherein a content of the condensate formed from the components (A) and (B) is preferably more than 1 mass%, more preferably 1.5 mass% or more, further more preferably 2.5 mass% or more, even more preferably 5 mass% or more, even more preferably 10 mass% or more, and preferably 80 mass% or less, more preferably 70 mass% or less, further more preferably 60 mass% or less, even more preferably 50 mass% or less, even more preferably 40 mass% or less.

<8> The fiber-treating agent according to any one of <1> to <7>, wherein the (C) water is a medium, and a content of the component (C) is preferably 10 mass% or more, more preferably 20 mass% or more, further more preferably 30 mass% or more, even more preferably 40 mass% or more, and preferably 99 mass% or less, more preferably 97 mass% or less, further more preferably 95 mass% or less, even more preferably 90 mass% or less.

<9> The fiber-treating agent according to any one of <1> to <8>, preferably further comprising the following component (D):

(D): an organic compound having a Hansen solubility parameter SP value of 16 $Mpa^{1/2}$ or more and 40 $Mpa^{1/2}$ or less (excluding organic salts and compounds having an aldehyde group and having a molecular weight of 150 or less).

<10> The fiber-treating agent according to <9>, wherein the component (D) is preferably at least one selected from the group consisting of a monohydric alcohol, a dihydric alcohol, a dihydric alcohol derivative, a polyhydric alcohol with a valence number of 3 or more, lactam, imidazolidinone, pyrimidinone, lactone, alkylene carbonate and a general-purpose organic solvent, more preferably at least one selected from the group consisting of a dihydric alcohol, lactam and imidazoline, further more preferably at least one selected from the group consisting of diethylene glycol, triethylene glycol, N-methylpyrrolidone, 1,3-dimethyl-2-imidazolidinone and DMDM hydantoin.

<11> The fiber-treating agent according to <9> or <10>, wherein Hansen solubility parameter SP value of the component (D) is preferably 35.8 $Mpa^{1/2}$ or less, more preferably 34.7 $Mpa^{1/2}$ or less, further more preferably 29.2 $Mpa^{1/2}$ or less, and preferably 17.8 $Mpa^{1/2}$ or more, more preferably 21.1 $Mpa^{1/2}$ or more, further more preferably 22.0 $Mpa^{1/2}$ or more.

<12> The fiber-treating agent according to any one of <1> to <11>, preferably further comprising a cationic surfactant.

<13> The fiber-treating agent according to <12>, wherein the cationic surfactant is preferably a long chain monoalkyl quaternary ammonium salt having one alkyl group having 8 to 24 carbon atoms and three alkyl groups having 1 to 4 carbon atoms, more preferably at least one selected from the group consisting of compounds of the following formula:

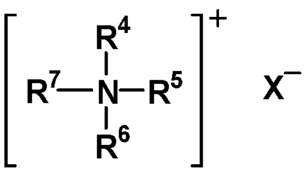

wherein $R^4$ is a saturated or unsaturated linear or branched alkyl group having 8 to 22 carbon atoms, $R^8$-CO-NH-$(CH_2)_m$- or $R^8$-CO-O-$(CH_2)_m$- ($R^8$ represents a saturated or unsaturated linear or branched alkyl chain having 7 to 21 carbon atoms, and m represents an integer of 1 to 4), $R^5$, $R^6$ and $R^7$ independently represent an alkyl group having 1 to 4 carbon atoms, or a hydroxyalkyl group having 1 to 4 carbon atoms, and $X^-$represents a hydrochloride ion, a bromide ion, a methosulfate ion or an ethosulfate ion;
further more preferably at least one selected from the group consisting of cetyltrimethylammonium chloride, myristyltrimethylammonium chloride, behentrimonium chloride, cetyltrimethylammonium bromide and stearamidopropyltrimonium chloride.

<14> The fiber-treating agent according to <12> or <13>, wherein a content of the cationic surfactant is preferably 0.05 mass% or more, more preferably 0.10 mass% or more, and preferably 10 mass% or less, more preferably 5 mass% or less.
<15> The fiber-treating agent according to any one of <1> to <14>, preferably further comprising silicone, more preferably one or more selected from the group consisting of dimethylpolysiloxane and amino acid-modified silicone.
<16> The fiber-treating agent according to <15>, wherein a content of the silicone is preferably 0.1 mass% or more, more preferably 0.2 mass% or more, further more preferably 0.5 mass% or more, and preferably 20 mass% or less, more preferably 10 mass% or less, further more preferably 5 mass% or less.
<17> The fiber-treating agent according to any one of <1> to <16>, preferably further comprising a cationic polymer.
<18> The fiber-treating agent according to <17>, wherein a content of the cationic polymer is preferably 0.001 mass% or more, more preferably 0.01 mass% or more, further more preferably 0.05 mass% or more, and preferably 20 mass% or less, more preferably 10 mass% or less.
<19> The fiber-treating agent according to any one of <1> to <18>, wherein a turbidity is preferably 500 NTU or less, more preferably 100 NTU or less, further more preferably 20 NTU or less.
<20> The fiber-treating agent according to any one of <1> to <19>, wherein a pH is preferably 6.0 or more, more preferably 6.5 or more, further more preferably 7.0 or more, and preferably 12.0 or less, more preferably 11.5 or less, further more preferably 11.0 or less.
<21> A method for producing a fiber-treating agent for treating human hair fibers separated from human heads, the method comprising heating a composition until its turbidity becomes 1,000 NTU or less, the composition comprising the following components (A) to (C):

(A): formaldehyde or a hydrate thereof;
(B): a condensate with a triazine derivative of formula (1):

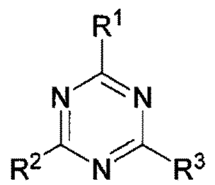

(1)

wherein $R^1$ to $R^3$ are the same or different, and each represent a hydrogen atom, a hydroxymethylamino group, an amino group, a hydroxy group, a halogen atom, a phenyl group, a linear or branched alkyl group or alkenyl group having 1 or more and 6 or less carbon atoms, or a linear or branched alkoxy group or alkenyloxy group having 1 or more and 6 or less carbon atoms; and
(C): water.

<22> The method for producing a fiber-treating agent according to <21>, wherein a heating temperature is preferably 85°C or higher, more preferably 88°C or higher, further more preferably 90°C or higher, and preferably 120°C or lower, more preferably 115°C or lower, further more preferably 110°C or lower.
<23> The method for producing a fiber-treating agent according to <21> or <22>, wherein a heating time is preferably 1 minute or more, more preferably 5 minutes or more, and preferably 20 minutes or less, more preferably 15 minutes

or less.

<24> The method for producing a fiber-treating agent according to any one of <21> to <23>, wherein a pH during heating is preferably 7.0 or more, more preferably 8.0 or more, further more preferably 9.0 or more, and preferably 13.0 or less, more preferably 12.0 or less, further more preferably 11.0 or less.

<25> A method for treating fibers, comprising the following step (i):

(i) immersing human hair fibers separated from human heads in the fiber-treating agent according to any one of <1> to <24> to perform treatment while maintaining a state in which a turbidity of the treating agent is 1,000 NTU or less.

<26> The method for treating fibers according to <25>, wherein preferably, the following step (0) is carried out before the step (i):

(0) heating the fiber-treating agent.

<27> The method for treating fibers according to <26>, wherein, assuming that T is a heating time until the treating agent has a turbidity of more than 1,000 NTU after the treating agent having a turbidity of 1,000 NTU or less is heated immediately after preparation, the heating time in the step (0) is preferably 0.2T or more, more preferably 0.3T or more, further more preferably 0.4T or more, and preferably 0.8T or less, more preferably 0.7T or less, further more preferably 0.6T or less.

<28> The method for treating fibers according to any one of <25> to <27>, wherein an amount of the fiber-treating agent in which the human hair fibers are immersed in the step (i) is preferably 2 or more, more preferably 3 or more, further more preferably 5 or more, even more preferably 10 or more, even more preferably 20 or more, and preferably 500 or less, more preferably 250 or less, further more preferably 100 or less, in terms of bath ratio to a mass of the human hair fibers (mass of fiber-treating agent/mass of human hair fibers).

<29> The method for treating fibers according to any one of <25> to <28>, wherein a temperature of the fiber-treating agent in the step (i) is preferably 30°C or higher, more preferably 40°C or higher, further more preferably 50°C or higher, and preferably lower than 95°C, more preferably 85°C or lower, further more preferably 75°C or lower.

<30> The method for treating fibers according to any one of <25> to <29>, wherein, assuming that T is a heating time until the treating agent has a turbidity of more than 1,000 NTU after the treating agent having a turbidity of 1,000 NTU or less is heated immediately after being prepared, an immersion time in the step (i) is preferably 0.3T or more, more preferably 0.4T or more, further more preferably 0.45T or more, and preferably 0.95T or less, more preferably 0.90T or less, further more preferably 0.85T or less.

<31> The method for treating fibers according to any one of <25> to <30>, wherein preferably, the following step (ii) is carried out after the step (i), and the step (i) and the step (ii) are repeated two or more times:

(ii) taking out the human hair fibers from the treating agent before the turbidity of the treating agent exceeds 1,000 NTU.

<32> The method for treating fibers according to <31>, wherein preferably, the following step (iii) is carried out after the step (ii):

(iii) rinsing the human hair fibers taken out.

<33> The method for treating fibers according to <32>, wherein preferably, the rinsing in the step (iii) is carried out using a rinsing composition containing a component D:

(D): an organic compound having a Hansen solubility parameter SP value of 16 $Mpa^{1/2}$ or more and 40 $Mpa^{1/2}$ or less (excluding organic salts and compounds having an aldehyde group and having a molecular weight of 150 or less).

<34> The method for treating fibers according to <33>, wherein the rinsing composition contains water in addition to the component (D), and the content of the component (D) in the rinsing composition is preferably 60 mass% or more, more preferably 80 mass% or more, further more preferably 95 mass% or more.

<35> The method for treating fibers according to any one of <25> to <34>, wherein preferably, the following step (iv) is further carried out after the steps (i) to (iii):

(iv) immersing human hair fibers in a post-cross-linking agent containing components (E) and (C):

(E): at least one formaldehyde derivative selected from the group consisting of formaldehyde, a hydrate of formaldehyde, glyoxylic acid, a hydrate of glyoxylic acid, a glyoxylic acid salt, glyoxal, a hydrate of glyoxal, glutaraldehyde, and a hydrate of glutaraldehyde; and

(C): water.

<36> The method for treating fibers according to <35>, wherein a content of the component (E) in the post-cross-linking agent is preferably 0.01 mass% or more, more preferably 0.1 mass% or more, further more preferably 1 mass% or more, and preferably 60 mass% or less, more preferably 40 mass% or less, further more preferably 20 mass% or less.

<37> The method for treating fibers according to <35> or <36>, wherein a temperature of the post-cross-linking

agent is preferably 40°C or higher, more preferably 50°C or higher, further more preferably 60°C or higher, and preferably lower than 100°C, more preferably 99°C or lower.
<38> The method for treating fibers according to any one of <25> to <37>, wherein preferably, the following step (v) is further carried out after the steps (i) to (iii) or after the step (iv):
(v) immersing human hair fibers in a surface finish agent (I) containing components (F) and (C):
(F): a resorcin derivative of formula (2):

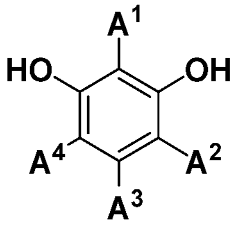

(2)

wherein

$A^1$ to $A^4$ are the same or different, and each represent a hydrogen atom, a hydroxy group, a halogen atom, a carboxyl group or a salt thereof, a sulfonic acid group or a salt thereof, a linear or branched alkyl group or alkenyl group having 1 to 6 carbon atoms, or a linear or branched alkoxy group or alkenyloxy group having 1 to 6 carbon atoms;
(C): water.

<39> The method for treating fibers according to <38>, wherein a content of the component (F) in the surface finish agent (I) is preferably 10 mass% or more, more preferably 20 mass% or more, further more preferably 30 mass% or more, even more preferably 40 mass% or more, even more preferably 50 mass% or more, and preferably 98 mass% or less, more preferably 97 mass% or less, further more preferably 95 mass% or less, even more preferably 90 mass% or less, even more preferably 80 mass% or less.
<40> The method for treating fibers according to <38> or <39>, wherein a temperature of the surface finish agent (I) is preferably 0°C or higher, more preferably 20°C or higher, further more preferably 40°C or higher, and preferably 80°C or lower, more preferably 60°C or lower.
<41> The method for treating fibers according to any one of <25> to <40>, wherein preferably, the step of immersing human hair fibers in a surface finish agent (II) containing the following components (G) and (C) is further carried out after the steps (i) to (iii), or after the step (iv), or after the step (v):
(G) an epoxyaminosilane copolymer which is a reaction product of the following compounds (a) to (d):

(a) polysiloxane having at least two oxiranyl groups or oxetanyl groups;
(b) polyether having at least two oxiranyl groups or oxetanyl groups;
(c) aminopropyltrialkoxysilane; and
(d) a compound selected from the group consisting of the following primary and secondary amines:

- primary amine: methylamine, ethylamine, propyleneamine, ethanolamine, isopropylamine, butylamine, isobutylamine, hexylamine, dodecylamine, oleylamine, aniline, aminopropyltrimethylsilane, aminopropyltriethylsilane, aminomorpholine, aminopropyldiethylamine, benzylamine, naphthylamine, 3-amino-9-ethylcarbazole, 1-aminoheptafluorohexane and 2,2,3,3,4,4,5,5,6,6,7,7,8,8,8-pentadecafluoro-1-octaneamine; and
- secondary amine: methylethylamine, methyloctadecylamine, diethanolamine, dibenzylamine, dihexylamine, dicyclohexylamine, piperidine, pyrrolidine phthalimide and polymer amine; and

(C) water.
<42> The method for treating fibers according to <41>, wherein preferably, the component (G) is polysilicone-29.
<43> The method for treating fibers according to <41> or <42>, wherein a content of the component (G) in the surface finish agent (II) is preferably 0.01 mass% or more, more preferably 0.05 mass% or more, further more preferably 0.10 mass% or more, further more preferably 0.20 mass% or more, and preferably 15.00 mass% or less, more preferably 10.00 mass% or less, further more preferably 8.00 mass% or less, further more preferably 6.00 mass% or less.
<44> A method for producing human hair fibers for hair ornament products, comprising the step of treating human hair fibers by the method for treating fibers according to any one of <25> to <43>.

<45> A method for producing a hair ornament product, comprising the step of treating human hair fibers by the method for treating fibers according to any one of <25> to <43>.

<46> A human hair fiber for hair ornament products, comprising a condensate formed from components (A) and (B):

(A): formaldehyde or a hydrate thereof; and
(B): a triazine derivative of formula (1):

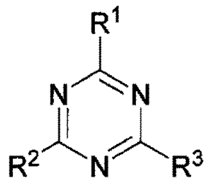

(1)

wherein $R^1$ to $R^3$ are the same or different, and each represent a hydrogen atom, a hydroxymethylamino group, an amino group, a hydroxy group, a halogen atom, a phenyl group, a linear or branched alkyl group or alkenyl group having 1 or more and 6 or less carbon atoms, or a linear or branched alkoxy group or alkenyloxy group having 1 or more and 6 or less carbon atoms.

<47> A hair ornament product having, as a constituent element, human hair fibers containing a condensate formed from components (A) and (B):

(A): formaldehyde or a hydrate thereof; and
(B): a triazine derivative of formula (1):

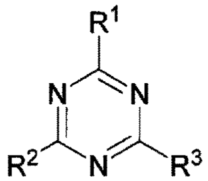

(1)

wherein $R^1$ to $R^3$ are the same or different, and each represent a hydrogen atom, a hydroxymethylamino group, an amino group, a hydroxy group, a halogen atom, a phenyl group, a linear or branched alkyl group or alkenyl group having 1 or more and 6 or less carbon atoms, or a linear or branched alkoxy group or alkenyloxy group having 1 or more and 6 or less carbon atoms.

<48> A human hair fiber-treating agent comprising the following components (A) to (C):

(A) formaldehyde: 1 to 15 mass%;
(B) one or more selected from the group consisting of melamine, monomethylol melamine, dimethylol melamine and trimethylol melamine: 5 to 30 mass%; and
(C) water.

<49> The human hair fiber-treating agent according to <48>, further comprising the following component (D):
(D) one or more selected from the group consisting of diethylene glycol, triethylene glycol, N-methylpyrrolidone, 1,3-dimethyl-2-imidazolidinone and DMDM hydantoin: 15 to 45 mass%

<50> A human hair fiber-treating agent kit comprising the human hair fiber-treating agent according to <48> or <49>, and a post-cross-linking agent containing components (E) and (C) and having a pH of 1.0 to 4.5:

(E) glyoxylic acid or formaldehyde: 1 to 20 mass%; and
(C) water: balance.

<51> The human hair fiber-treating agent kit according to <50>, preferably further comprising a surface finish agent (II) containing components (G) and (C):

(G) polysilicone-29: 0.1 to 15 mass%; and
(C) water: balance.

<52> A human hair fiber-treating agent kit comprising the human hair fiber-treating agent according to <48> or <49>, and a surface finish agent (II) containing components (G) and (C):

(G) polysilicone-29: 0.1 to 15 mass%; and
(C) water: balance.

## Examples

### Examples 1 to 10 and Comparative Example 1

Using compositions whose formulations are shown in Tables 1 and 2, human hair fibers were treated by the following method, and various properties were evaluated. The pH of each composition was measured with the prepared composition directly applied to a pH meter (F-52 manufactured by HORIBA, Ltd.) at room temperature (25°C). The turbidity of the composition was measured with a fiber-treating agent directly placed in a measurement cell (ϕ25 × 60 mm borosilicate glass) of a digital turbidimeter (manufactured by AS ONE Corporation/model: TB700/measurement method: equivalent to ISO 7027, Nephelometry (90°)/light source: infrared emitting diode (850 nm)/detector: crystal silicon solar cell module) at room temperature (25°C).

<Treatment method>

1. A 22 cm-long tress with 0.5 g of human hair fibers (cut from a common marketed 100% human hair wig) was immersed in a container containing 40 g of the fiber-treating agent, the opening of the container was closed, the container was immersed together with its contents in a water bath (manufacturer: TOYO SEISAKUSHO, Ltd./Model: TBS221FA) at a predetermined temperature, and heating was performed for a predetermined time. The heating time here (step (i)) was fixed to a time equivalent to about 0.45T (T is as described above).
For Examples 6 and 7, heating was performed for a time equivalent to about 0.30T (step (0)) before the above-described procedure 1.
2. The container containing the tress was taken out from the water bath, and brought back to room temperature.
3. The tress was taken out from the container, immersed in 50 g of triethylene glycol for 30 seconds, then rinsed with running tap water at 30°C for 30 seconds, lathered with evaluating shampoo for 60 seconds, rinsed with running tap water at 30°C for 30 seconds, and lightly drained with a towel, and the tress was blown by a hot air dryer (Nobby White NB 3000 manufactured by TESCOM Company) while being combed. At this time, the tress remained straight.

<Post-cross-linking treatment> (Example 7 only)

4. The tress subjected to the above-described treatment was immersed in a container containing 40 g of a post-cross-linking agent, the opening of the container was closed, the container was immersed together with its contents in a water bath (manufacturer: TOYO SEISAKUSHO, Ltd./Model: TBS221FA) at a predetermined temperature, and heating was performed for a predetermined time.
5. The container containing the tress was taken out from the water bath, and brought back to room temperature.
6. The tress was taken out from the container, rinsed with running tap water at 30°C for 30 seconds, lathered with evaluating shampoo for 60 seconds, rinsed with running tap water at 30°C for 30 seconds, and lightly drained with a towel, and the tress was blown by a hot air dryer (Nobby White NB 3000 manufactured by TESCOM Company) while being combed. At this time, the tress remained straight.

[Evaluation method]

<Maintenance of stretchability (tenacity) during fiber tensioning>

As an index of stretchability (tenacity) during fiber tensioning, an average breaking elongation, that is, an average value in evaluation on a plurality of fibers (ten fibers) for the percentage by which the fiber was stretched by tensioning with respect to the original fiber length when rupture occurred was used. The evaluation was performed in the following procedure using a tress immediately after treatment performed as described in <Treatment method> above (<Treatment method> and <Post-cross-linking> for Example 7).

1. Ten fibers were cut from the root of the tress. A 3 cm fiber fragment was taken from near the center between the root and the hair tip of each fiber, so that a total of ten 3 cm hair fragments were obtained. These hair fragments were left to stand in a chamber at 20°C and 65% RH for 12 hours or more.

2. The fiber fragment was set in "MTT690 Miniature Tensile Tester" manufactured by DIA-STRON Limited, automatic measurement was started, and an average breaking elongation was determined when the fiber was in a dry state. A higher numerical value shows higher stretchability, better tenacity and better durability.

(Evaluation Criteria)

Differences in stretchability (tenacity) during fiber tensioning in comparison to the untreated hair of Comparative Example 1 are shown as X (%) in the table. There may be both positive and negative numerical values as X. A larger numerical value of X when X is positive or a smaller absolute value of X when X is negative shows higher stretchability, better tenacity and better durability.

That is, the degree of decrease (X%) in average breaking elongation of the treated tress (B%) with respect to an untreated state when the average breaking elongation during fiber tensioning in an intact (untreated) state at the time of being cut from the marketed product (A%) is used as a reference is shown in the table as the "amount of decrease of stretchability (tenacity) during fiber tensioning as X (%)".

$$X\ (\%) = B\ (\%) - A\ (\%)$$

<Achievement of fiber tensile elastic modulus close to that of normal hair>

Evaluation of the fiber tensile elastic modulus was performed using a tress immediately after treatment performed as described in <Treatment method> above. As a numerical value, an average value in evaluation on a plurality of fibers (ten fibers) was used. The evaluation was performed in the following procedure.

1. Ten fibers were cut from the root of the tress. A 3 cm fiber fragment was taken from near the center between the root and the hair tip of each fiber, so that a total of ten 3 cm hair fragments were obtained. These hair fragments were left to stand in a chamber at 20°C and 65% RH for 12 hours or more.
2. The fiber fragment was set in "MTT690 Miniature Tensile Tester" manufactured by DIA-STRON Limited, automatic measurement was started, and a fiber tensile elastic modulus was determined when the fiber was in a dry state.
3. As an index of fiber tensile elongation, fiber tensile elasticity normalization ratio $E = (E_1/E_0) \times 100$ [%] is defined, where $E_1$ is a fiber tensile elastic modulus determined by the above-described procedures 1 and 2, and Eo is a fiber elastic modulus similarly determined using normal hair (*). An E value close to 100% shows that the fiber is normalized, has natural tension and stiffness, and is resistant to stretching by an external force and excellent in durability.

* As normal hair, ten hairs of 30 cm from the root portion, which were taken from a 20-year-old Japanese woman who had not undergone chemical treatment, were used, and in the same manner as described above, fiber fragments were taken and measurement was performed. However, the human hair fibers (untreated hair) used here had an elastic modulus lower than that of normal hair as shown in Comparative Example 1, and this may be because these human hair fibers were taken from a marketed wig, and were accordingly already damaged.

<Shaping>

Evaluation of heat shape memory ability was performed using a tress immediately after treatment performed as described in <Treatment method> above (<Treatment method> and <Post-cross-linking> for Example 7).

• I: Shaping (curl)

1. A 22 cm-long tress with 0.5 g of human hair fibers was wetted with tap water at 30°C for 30 seconds, and the wet tress was then wound around a plastic rod having a diameter of 14 mm, and fixed with a clip.
2. The tress wound around the rod was immersed together with the rod in a water bath (manufacturer: TOYO SEISAKUSHO, Ltd./Model: TBS221FA) at 60°C, and heated for 1 minute.
3. The tress was taken out from the water bath, and immersed in water at 25°C for 1 minute to be brought back to room temperature.
4. The tress was removed from the rod, combed three times, and then hung, and photographed right from the side.

(Evaluation Criteria)

The curling-up ratio = ratio of decrease in tress length (I) (%) determined from the following expression, where Lo is an untreated tress length (22 cm) and L is a treated tress length, was defined as curling strength.

$$I = [(L_0 - L)/L_0] \times 100$$

A : $20 \leq I$
B : $15 \leq I < 20$
C : $10 \leq I < 15$
D : $5 \leq I < 10$
E : $I < 5$

• II: Reshaping (straight)

1. The tress evaluated in I was combed to eliminate entanglement, and a flat iron (manufactured by Miki Denki Sangyo K.K./Model: AHI-938) at a measured temperature of 180°C was then slid over the tress six times at a rate of 5 cm/sec.
2. The tress was rinsed with running tap water at 30°C for 30 seconds, lathered with evaluating shampoo for 60 seconds, then rinsed with running tap water at 30°C for 30 seconds, and dried with a towel.
3. The tress was dried (without using a dryer) while being vibrated so as to obtain a natural shape as human hair fibers, and was combed, then hung, and visually observed right from the side.

(Evaluation Criteria)

A: No curl, and completely straightened.
B: Less curled than before flat iron treatment, but not completely straightened.
C: Curl and shape sustained as before treatment.

<III: Re-reshaping (Curl)>

1. The tress evaluated in II was wetted with tap water at 30°C for 30 seconds, and the wet tress was then wound around a plastic rod having a diameter of 14 mm, and fixed with a clip.
2. The tress wound around the rod was immersed together with the rod in a water bath (manufacturer: TOYO SEISAKUSHO, Ltd./Model: TBS221FA) at 60°C, and heated for 1 minute.
3. The tress was taken out from the water bath, and immersed in water at 25°C for 1 minute to be brought back to room temperature.
4. The tress was removed from the rod, combed three times, and then hung, and photographed right from the side.

(Evaluation Criteria)

The curling-up ratio = ratio of decrease in tress length (I) (%) determined from the following expression, where Lo is an untreated tress length (22 cm) and L is a treated tress length, was defined as curling strength.

$$I = [(L_0 - L)/L_0] \times 100$$

A: $20 \leq I$

B: $15 \leq I < 20$

C: 10 ≤ I < 15

D: 5 ≤ I < 10

E: I < 5

<Formulation of evaluating shampoo>

| Component | (mass%) |
|---|---|
| sodium laureth sulfate | 15.5 |
| lauramide DEA | 1.5 |
| sodium benzoate | 0.5 |
| EDTA-2Na | 0.3 |
| phosphoric acid | amount required to adjust pH to 7 |
| ion-exchange water | balance |
| total | 100 |

<Surface feel quality>

For evaluation of the feel, five skilled panelists performed evaluation on the basis of the following criteria for feel smoothness when the tress immediately after evaluation in <Shape sustainability> was touched by hand, and a total value for the five panelists was taken as an evaluation result.

(Evaluation Criteria)

5: Much smoother hand feel over untreated fibers.
4: Smoother hand feel over untreated fibers.
3: Slightly smoother hand feel over untreated fibers.
2: Comparable in hand feel to untreated fibers.
1: Rougher, more frictional and poorer in hand feel than untreated fibers.

[Table 1]

| | | | Example 1 | Example 2 | Example 3 | Example 4 | Example 5 | Example 6 | Example 7 | Comparative Example 1 |
|---|---|---|---|---|---|---|---|---|---|---|
| Treating agent (mass%) (*1) | (A) | Formaldehyde | 5.0 | 4.2 | 3.5 | 3.5 | 3.5 | 3.5 | 3.5 | Not treated |
| | (B) | Melamine | 14.0 | 14.0 | 14.0 | 14.0 | 14.0 | 14.0 | 14.0 | |
| | (C) | Water | Balance | Balance | Balance | Balance | Balance | Balance | Balance | |
| | (D) | Triethylene glycol | 30.7 | 30.7 | 30.7 | 30.7 | - | 30.7 | 30.7 | |
| | pH adjuster | Hydrochloric acid or sodium hydroxide | *2 | *2 | *2 | *2 | *2 | *2 | *2 | |
| | Total | | 100 | 100 | 100 | 100 | 100 | 100 | 100 | |
| | pH(25°C) | | 9.0 | 9.0 | 9.0 | 9.0 | 9.0 | 9.0 | 9.0 | |
| | Molar ratio (A)/(B) | | 1.5 | 1.25 | 1.05 | 1.05 | 1.05 | 1.05 | 1.05 | |
| | Heating for producing treating agent (turbidity: 1,000 or less) | | 90°C 10 min | 90°C 10 min | 90°C 10 min | 90°C 10 min | 90°C 10 min | 90°C 10 min | 90°C 10 min | |
| Treatment method | Heating in step (0) (heat treating agent having a turbidity of 1,000 or less before treatment of fibers) | | None | None | None | None | None | 90°C 20 min | 90°C 20 min | |
| | Bath ratio (mass ratio of treating agent to human hair fibers) | | 80 | 80 | 80 | 80 | 80 | 80 | 80 | |
| | Heating condition | | 90°C 30 min ×1 | 90°C 30 min ×1 | 90°C 30 min ×1 | 65°C 120 min ×1 | 65°C 60 min ×1 | 65°C 120 min ×1 | 65°C 120 min ×1 | |
| | Action for preventing formulation turbidity from exceeding 1,000 NTU during immersion of fibers. | | Take out fibers | Take out fibers | Take out fibers | Take out fibers | Take out fibers | Take out fibers | Take out fibers | |
| | Solvent used for washing fibers at the time of takeout or at the time of agent replacement (immersion and washing at bath ratio of 1 : 100) | | Triethylene glycol | Triethylene glycol | Triethylene glycol | Triethylene glycol | Triethylene glycol | Triethylene glycol | Triethylene glycol | |

(continued)

| | | | Example | | | | | | | Comparative Example |
|---|---|---|---|---|---|---|---|---|---|---|
| | | | 1 | 2 | 3 | 4 | 5 | 6 | 7 | 1 |
| Post-cross-linking agent (mass%) | (E) | Glyoxylic acid | Not treated | Not treated | Not treated | Not treated | Not treated | Not treated | 10.0 | |
| | (C) | Water | | | | | | | Balance | |
| | pH adjuster | Sodium hydroxide | | | | | | | *2 | |
| | Total | | | | | | | | 100 | |
| | pH(25°C) | | | | | | | | 5.0 | |
| Treatment method | Heating condition | | | | | | | | 70°C 1 h × 1 | |
| Effect | Durability improvement | Amount of decrease in stretchability (tenacity)during fiber tensioning [%] | -17.8 | -14.1 | -13.5 | -8.3 | -15.4 | -5.9 | -5.6 | (Reference) |
| | | Fiber tensile elasticity normalization ratio [%] | 95 | 99 | 95 | 98 | 105 | 101 | 98 | 88 |
| | Shaping | I: Shaping (curl) | B | A | B | C | A | B | A | E |
| | | II: Reshaping (straight) | A | A | A | A | A | A | A | - |
| | | III: Re-reshaping (curl) | B | A | B | C | A | B | A | - |
| | Surface feel quality | | 15 | 16 | 17 | 19 | 15 | 18 | 20 | 10 |

*1: Composition before heating
*2: Amount of pH adjustment

[Table 2]

| | | | Example 8 | Example 9 | Example 10 |
|---|---|---|---|---|---|
| Treating agent (mass%) (*1) | (A) | Formaldehyde | 0.2 | 0.5 | 2.0 |
| | (B) | Melamine | 0.8 | 2.0 | 8.0 |
| | (C) | Water | Balance | Balance | Balance |
| | (D) | Triethylene glycol | 30.7 | 30.7 | 30.7 |
| | pH adjuster | Hydrochloric acid or sodium hydroxide | *2 | *2 | *2 |
| | Total | | 100 | 100 | 100 |
| | pH(25°C) | | 9.0 | 9.0 | 9.0 |
| | Molar ratio (A)/(B) | | 1.05 | 1.05 | 1.05 |
| | Heating for producing treating agent (turbidity: 1,000 or less) | | 90°C 10 min | 90°C 10 min | 90°C 10 min |
| Treatment method | Heating in step (0) (heat treating agent having a turbidity of 1,000 or less before treatment of fibers) | | None | None | None |
| | Bath ratio (mass ratio of treating agent to human hair fibers) | | 80 | 80 | 80 |
| | Heating condition | | 65°C 240 min ×1 | 65°C 240 min ×1 | 65°C 240 min ×1 |
| | Action for preventing formulation turbidity from exceeding 1,000 NTU during immersion of fibers. | | Take out fibers | Take out fibers | Take out fibers |
| | Solvent used for washing fibers at the time of takeout or at the time of agent replacement (immersion and washing at bath ratio of 1 : 100) | | Triethylene glycol | Triethylene glycol | Triethylene glycol |
| Effect | Durability improvement | Amount of decrease in stretchability (tenacity) during fiber tensioning [%] | -0.2 | -2.2 | -5.6 |
| | | Fiber tensile elasticity normalization ratio [%] | 90 | 92 | 96 |
| | Shaping | I: Shaping (curl) | D | B | A |
| | | II: Reshaping (straight) | A | A | A |
| | | III: Re-reshaping (curl) | D | B | A |
| | Surface feel quality | | 18 | 17 | 16 |

*1: Composition before heating
*2: Amount of pH adjustment

Example 11, Comparative Example 2

Table 3 shows Example in which step (i) and step (ii) were repeated a plurality of times to the extent that the turbidity of the composition did not exceed 1,000 NTU and Comparative Example in which heating was continued even after the turbidity of the composition exceeded 1,000 NTU in the middle of the step (i), in "1" of <Treatment method> above.

In Example 11, human hair fibers were treated in the same manner as in <Treatment method> above except that an operation was carried out in which human hair fibers were taken out before the turbidity of the composition exceeded 1,000 NTU in the step (i), the composition was replaced by a new similar composition, and similar heating treatment was performed, and the human hair fibers were taken out before the turbidity of the composition exceeded

1,000 NTU (a total of two heating treatments were performed).

In Comparative Example 2, human hair fibers were treated in the same manner as in <Treatment method> above except that even after the turbidity of the composition exceeded 1,000 NTU in the step (i), heating was continued while the composition remained cloudy.

[Table 3]

| | | | Example | Comparative Example |
|---|---|---|---|---|
| | | | 11 | 2 |
| Treating agent (mass%) (*1) | (A) | Formaldehyde | 3.5 | 3.5 |
| | (B) | Melamine | 14.0 | 14.0 |
| | (C) | Water | Balance | Balance |
| | (D) | Triethylene glycol | 30.7 | 30.7 |
| | pH adjuster | Hydrochloric acid or sodium hydroxide | *2 | *2 |
| | Total | | 100 | 100 |
| | pH(25°C) | | 9.0 | 9.0 |
| | Molar ratio (A)/(B) | | 1.05 | 1.05 |
| | Heating for producing treating agent (turbidity: 1,000 or less) | | 90°C 10 min | 90°C 10 min |
| Treatment method | Heating in step (0) (heat treating agent having a turbidity of 1,000 or less before treatment of fibers) | | None | None |
| | Bath ratio (mass ratio of treating agent to human hair fibers) | | 80 | 80 |
| | Heating condition | | 65°C 120 min ×2 | 65°C 240 min ×1 |
| | Action for preventing formulation turbidity from exceeding 1,000 NTU during immersion of fibers. | | Agent replacement | None (white turbidness) |
| | Solvent used for washing fibers at the time of takeout or at the time of agent replacement (immersion and washing at bath ratio of 1 : 100) | | Triethylene glycol | Triethylene glycol |
| Effect | Durability improvement | Amount of decrease in stretchability (tenacity) during fiber tensioning [%] | -15.1 | -21.4 |
| | | Fiber tensile elasticity normalization ratio [%] | 96 | 112 |
| | Shaping | I: Shaping (curl) | A | A |
| | | II: Reshaping (straight) | A | A |
| | | III: Re-reshaping (curl) | A | A |
| | Surface feel quality | | 15 | 8 |
| *1: Composition before heating<br>*2: Amount of pH adjustment | | | | |

Comparative Example 2, in which the amount of decrease in stretchability (tenacity) during fiber tensioning was -21.4 and the surface feel quality was 8, was inferior to Example 11. The fiber tensile elasticity normalization ratio is 112, and there is a significant difference in comparison to the most preferred value of 100. This shows that a hard resin layer was formed on the surface, so that the hair excessively hardened as compared to normal hair.

Example 12 (surface finish treatment)

The human hair fibers treated in Example 4 were treated with a surface finish agent shown in Table 4, and various properties were evaluated.

<Treatment method>

1. The tress was immersed in a container containing 40 g of the surface finish agent, and left to stand at room temperature for 30 minutes.
2. The tress was taken out from the container, and dried for 5 minutes with a household centrifugal dryer (Ultrafast Dryer Powerful Spin Dry APD-6.0 manufactured by ALUMIS CO., LTD.) (spin coating method).
3. The tress was taken out from the dryer, and heated for 3 hours in an oven (stainless steel forced circulation dryer; SOFW-450 manufactured by AS ONE Corporation) set at 60°C.
4. The tress was taken out from the oven, and brought back to room temperature.
5. The tress was rinses with running water at 30°C for 30 seconds, and lightly drained with a towel, and the tress was then blown by a hot air dryer (Nobby White NB 3000 manufactured by TESCOM Company) while being combed.

[Table 4]

| | | | Example 12 |
|---|---|---|---|
| Surface finish agent (mass%) | (G) | Polysilicone-29 | 5.0 |
| | (C) | Water | Balance |
| | Total | | 100 |
| | pH(25°C) | | 4.0 |
| Treatment method | Surface finish treatment method | | Spin coating |
| Effect | Durability improvement | Amount of decrease in stretchability (tenacity) during fiber tensioning [%] | -8.8 |
| | Surface feel quality | | 24 |

**Claims**

**1.** A fiber-treating agent for treating human hair fibers separated from human heads, the fiber-treating agent comprises a condensate formed from the following components (A) and (B), and a component (C), and has a turbidity of 1,000 NTU or less:

(A): formaldehyde or a hydrate thereof;
(B): a triazine derivative of formula (1):

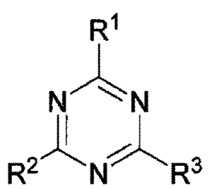

(1)

wherein $R^1$ to $R^3$ are the same or different, and each represent a hydrogen atom, a hydroxymethylamino group, an amino group, a hydroxy group, a halogen atom, a phenyl group, a linear or branched alkyl group or alkenyl group having 1 or more and 6 or less carbon atoms, or a linear or branched alkoxy group or alkenyloxy group having 1 or more and 6 or less carbon atoms; and
(C): water.

**2.** The fiber-treating agent according to claim 1, wherein a content of a constituent element derived from the component (A) in the fiber-treating agent is 0.1 mass% or more and 60 mass% or less.

**3.** The fiber-treating agent according to claim 1 or 2, wherein a content of a constituent element derived from the component (B) in the fiber-treating agent is 0.1 mass% or more and 60 mass% or less.

**4.** The fiber-treating agent according to any one of claims 1 to 3, wherein a total content of the constituent element derived from the component (A) and the constituent element derived from the component (B) in the fiber-treating agent is 0.1 mass% or more and 80 mass% or less.

**5.** The fiber-treating agent according to any one of claims 1 to 4, wherein a molar ratio of the constituent element derived from the component (A) to the constituent element derived from the component (B), (A)/(B), is less than 5.

**6.** The fiber-treating agent according to any one of claims 1 to 5, further comprising the following component (D): (D): an organic compound having a Hansen solubility parameter SP value of 16 $Mpa^{1/2}$ or more and 40 $Mpa^{1/2}$ or less (excluding organic salts and compounds having an aldehyde group and having a molecular weight of 150 or less).

**7.** A method for producing a fiber-treating agent for treating human hair fibers separated from human heads, the method comprising heating a composition until its turbidity becomes 1,000 NTU or less, the composition comprising the following components (A) to (C):

(A): formaldehyde or a hydrate thereof;
(B): a condensate with a triazine derivative of formula (1):

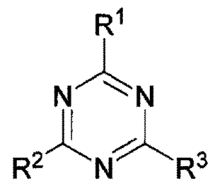 (1)

wherein $R^1$ to $R^3$ are the same or different, and each represent a hydrogen atom, a hydroxymethylamino group, an amino group, a hydroxy group, a halogen atom, a phenyl group, a linear or branched alkyl group or alkenyl group having 1 or more and 6 or less carbon atoms, or a linear or branched alkoxy group or alkenyloxy group having 1 or more and 6 or less carbon atoms; and
(C): water.

**8.** A method for treating fibers, comprising the following step (i):

(i) immersing human hair fibers separated from human heads in the fiber-treating agent according to any one of claims 1 to 6 to perform treatment while maintaining a state in which a turbidity of the treating agent is 1,000 NTU or less.

**9.** The method for treating fibers according to claim 8, wherein the following step (0) is carried out before the step (i): (0) heating the fiber-treating agent.

**10.** The method for treating fibers according to claim 9, wherein, assuming that T is a heating time for heating the treating agent having a turbidity of 1,000 NTU or less immediately after preparation until the turbidity exceeds 1,000 NTU again, a heating time in the step (0) is 0.2T or more and 0.8T or less.

**11.** The method for treating fibers according to any one of claims 8 to 10, wherein the following step (ii) is carried out after the step (i), and wherein the step (i) and the step (ii) are repeated two or more times:
(ii) taking out the human hair fibers from the treating agent before the turbidity of the treating agent exceeds 1,000 NTU.

**12.** The method for treating fibers according to claim 11, wherein the following step (iii) is carried out after the step (ii): (iii) rinsing the human hair fibers taken out.

**13.** The method for treating fibers according to claim 12, wherein the rinsing in the step (iii) is performed using a

composition containing a component (D):
(D): an organic compound having a Hansen solubility parameter SP value of 16 $Mpa^{1/2}$ or more and 40 $Mpa^{1/2}$ or less (excluding organic salts and compounds having an aldehyde group and having a molecular weight of 150 or less).

**14.** The method for treating fibers according to any one of claims 8 to 13, wherein the following step (iv) is further carried out after the steps (i) to (iii):
(iv) immersing human hair fibers in a post-cross-linking agent containing components (E) and (C):

(E): at least one formaldehyde derivative selected from the group consisting of formaldehyde, a hydrate of formaldehyde, glyoxylic acid, a hydrate of glyoxylic acid, a glyoxylic acid salt, glyoxal, a hydrate of glyoxal, glutaraldehyde, and a hydrate of glutaraldehyde; and
(C): water.

**15.** The method for treating fibers according to any one of claims 8 to 14, wherein the step of immersing human hair fibers in a surface finish agent containing the following components (G) and (C) is further carried out after the steps (i) to (iii) or the step (iv):

(G) an epoxyaminosilane copolymer which is a reaction product of the following compounds (a) to (d):

(a) polysiloxane having at least two oxiranyl groups or oxetanyl groups;
(b) polyether having at least two oxiranyl groups or oxetanyl groups;
(c) aminopropyltrialkoxysilane; and
(d) a compound selected from the group consisting of the following primary and secondary amines:

- primary amine: methylamine, ethylamine, propyleneamine, ethanolamine, isopropylamine, butylamine, isobutylamine, hexylamine, dodecylamine, oleylamine, aniline, aminopropyltrimethylsilane, aminopropyltriethylsilane, aminomorpholine, aminopropyldiethylamine, benzylamine, naphthylamine, 3-amino-9-ethylcarbazole, 1-aminoheptafluorohexane and 2,2,3,3,4,4,5,5,6,6,7,7,8,8,8-pentadecafluoro-1-octaneamine; and
- secondary amine: methylethylamine, methyloctadecylamine, diethanolamine, dibenzylamine, dihexylamine, dicyclohexylamine, piperidine, pyrrolidine phthalimide and polymer amine; and

(C) water.

**16.** A method for producing human hair fibers for hair ornament products, comprising the step of treating human hair fibers by the method for treating fibers according to any one of claims 8 to 15.

**17.** A method for producing a hair ornament product, comprising the step of treating human hair fibers by the method for treating fibers according to any one of claims 8 to 15.

**18.** A human hair fiber for hair ornament products, comprising a condensate formed from components (A) and (B):

(A): formaldehyde or a hydrate thereof; and
(B): a triazine derivative of formula (1):

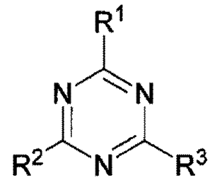

**(1)**

wherein $R^1$ to $R^3$ are the same or different, and each represent a hydrogen atom, a hydroxymethylamino group, an amino group, a hydroxy group, a halogen atom, a phenyl group, a linear or branched alkyl group or alkenyl group having 1 or more and 6 or less carbon atoms, or a linear or branched alkoxy group or alkenyloxy group having 1 or more and 6 or less carbon atoms.

**19.** A hair ornament product comprising, as a constituent element, human hair fibers containing a condensate formed

from components (A) and (B):

(A): formaldehyde or a hydrate thereof; and
(B): a triazine derivative of formula (1):

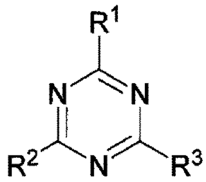

(1)

wherein $R^1$ to $R^3$ are the same or different, and each represent a hydrogen atom, a hydroxymethylamino group, an amino group, a hydroxy group, a halogen atom, a phenyl group, a linear or branched alkyl group or alkenyl group having 1 or more and 6 or less carbon atoms, or a linear or branched alkoxy group or alkenyloxy group having 1 or more and 6 or less carbon atoms.

INTERNATIONAL SEARCH REPORT

International application No.
PCT/JP2021/047434

**A. CLASSIFICATION OF SUBJECT MATTER**

***A41G 3/00***(2006.01)i; ***D06M 13/12***(2006.01)i; ***D06M 15/423***(2006.01)i; ***D06M 15/643***(2006.01)i
FI: A41G3/00 B; D06M13/12; D06M15/643; D06M15/423

According to International Patent Classification (IPC) or to both national classification and IPC

**B. FIELDS SEARCHED**

Minimum documentation searched (classification system followed by classification symbols)

A41G3/00; D06M13/12; D06M15/423; D06M15/643

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y<br>X | WO 2019/159867 A1 (KAO CORP.) 22 August 2019 (2019-08-22)<br>paragraphs [0007]-[0207] | 1-17<br>18-19 |
| Y | JP 11-172073 A (BORDEN CHEMICAL INC.) 29 June 1999 (1999-06-29)<br>paragraphs [0026]-[0042] | 1-17 |
| Y | WO 01/06045 A1 (KANEKA CORP.) 25 January 2001 (2001-01-25)<br>pp. 6, 7 | 6, 8-17 |
| Y | WO 2019/159866 A1 (KAO CORP.) 22 August 2019 (2019-08-22)<br>paragraphs [0121]-[0123] | 13-17 |

☐ Further documents are listed in the continuation of Box C.　☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 February 2022** | **22 February 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2021/047434**

| Patent document cited in search report | Publication date (day/month/year) | Patent family member(s) | Publication date (day/month/year) |
|---|---|---|---|
| WO 2019/159867 A1 | 22 August 2019 | US 2021/0115623 A1<br>paragraphs [0002]-[0342]<br>CN 111757684 A | |
| JP 11-172073 A | 29 June 1999 | US 5952440 A<br>column 5, 6<br>CN 1224736 A | |
| WO 01/06045 A1 | 25 January 2001 | US 6713537 B1<br>column 5<br>CN 1420949 A | |
| WO 2019/159866 A1 | 22 August 2019 | US 2021/0059336 A1<br>paragraphs [0146]-[0157]<br>CN 111757683 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005037000 A
- JP 2007177370 A
- JP 2019143282 A


### Non-patent literature cited in the description


- Parameters: A User's handbook. CRC Press, 2007